(19) Europäisches Patentamt
European Patent Office
Office européen des brevets

(11) EP 3 042 899 A1

(12) **EUROPEAN PATENT APPLICATION**
published in accordance with Art. 153(4) EPC

(43) Date of publication:
**13.07.2016 Bulletin 2016/28**

(21) Application number: **14842654.7**

(22) Date of filing: **01.09.2014**

(51) Int Cl.:
***C07D 401/10*** (2006.01) ***C07D 401/14*** (2006.01)
***A61K 31/506*** (2006.01) ***A61K 31/5377*** (2006.01)
***A61P 19/08*** (2006.01) ***A61P 29/00*** (2006.01)
***A61P 35/00*** (2006.01) ***A61P 37/06*** (2006.01)

(86) International application number:
**PCT/JP2014/072917**

(87) International publication number:
**WO 2015/033888 (12.03.2015 Gazette 2015/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **03.09.2013 JP 2013182310**

(71) Applicant: **Carna Biosciences, Inc.**
**Kobe-shi, Hyogo 650-0047 (JP)**

(72) Inventors:
- **KAWAHATA, Wataru**
**Kobe-shi**
**Hyogo 6500047 (JP)**
- **ASAMI, Tokiko**
**Kobe-shi**
**Hyogo 6500047 (JP)**
- **SAWA, Masaaki**
**Kobe-shi,**
**Hyogo 6500047 (JP)**
- **IRIE, Takayuki**
**Kobe-shi**
**Hyogo 6500047 (JP)**

(74) Representative: **Carpmaels & Ransford LLP**
**One Southampton Row**
**London WC1B 5HA (GB)**

(54) **NOVEL 2,6-DIAMINOPYRIMIDINE DERIVATIVE**

(57) To provide a novel 2,6-diaminopyrimidine derivative by the following formula (I):
A 2,6-diaminopyrimidine derivative is represented by the formula (I):

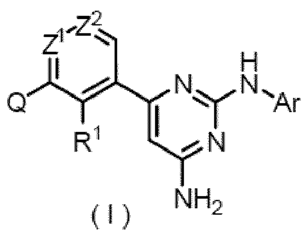

( I )

wherein
$R^1$ represents a substituted or unsubstituted lower alkyl group, or a substituted or unsubstituted alkoxy group,
Ar represents a substituted or unsubstituted aryl group, or a substituted or unsubstituted heteroaryl group,
$Z^1$ and $Z^2$ represent carbon atoms, or either 1 or 2 of the $Z^1$ and $Z^2$ represent nitrogen atoms,
Q is selected from a structure (a) or (b) described below:



Printed by Jouve, 75001 PARIS (FR)

(Cont. next page)

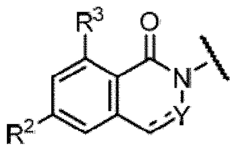
(a)

,

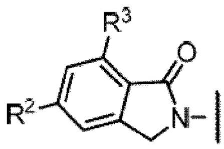
(b)

$R^2$ represents a substituted or unsubstituted lower alkyl group, or a substituted or unsubstituted cycloalkyl group,
$R^3$ represents a hydrogen atom or a halogen atom,
Y represents a nitrogen atom or a carbon atom, and
the bond drawn with a dotted line parallel to a solid line on structure (a) represents either double bond or single bond.

## Description

TECHNICAL FIELD

The present invention relates to a pharmaceutical, and particularly to a novel 2,6-diaminopyrimidine derivative having a BTK inhibitory effect, or a pharmaceutically acceptable salt thereof.

BACKGROUND ART

Bruton's tyrosine kinase (BTK) is a member of the Tec family of non-receptor tyrosine kinases, and is an important signaling enzyme which is expressed in all hematopoietic cell types except for T lymphocytes and natural killer cells. BTK is an important control factor associated with survival, differentiation, proliferation and activation of B-cells, and takes an important role in signaling of B-cells (Non-Patent Documents 1 and 2). A B-cell receptor (BCR) of the cell surface signals into cells through BTK existing in the downstream of BCR and, therefore, it is considered that abnormal activation of the signaling pathway of B-cells accelerates proliferation and survival of cancer cells of B-cell lymphoma, chronic lymphocytic leukemia and the like (Non-Patent Document 3). It is known that BTK also plays an important role in the signal pathway of a large number of other cells, and it is said that BTK is involved in allergic diseases, self-immune diseases, inflammatory diseases and the like (Non-Patent Document 1). For example, it is known that BTK plays an important role for signaling of a high affinity IgE receptor (FcεRI) in mast cells, and in BTK-deficient mast cells, degranulation and the production of proinflammatory cytokines are decreasing (Non-Patent Document 4). It is suggested that BTK is involved in systemic lupus erythematosus (SLE) in a test of a BTK-deficient mouse (Non-Patent Document 5). Furthermore, the BTK mutant mouse exhibits resistance to the onset of collagen-induced arthritis (Non-Patent Document 6). Therefore, the compound having a BTK inhibitory activity is useful for the treatment of diseases which are involved in BTK signaling, for example, cancer, B-cell lymphoma, and chronic lymphocytic leukemia, and is also useful for the treatment of allergic diseases, self-immune diseases and inflammatory diseases.

Although a compound having a BTK inhibitory effect has hitherto been reported, and a compound that retains pyrimidine ring structure having a BTK inhibitory effect has been reported in some patents (Patent Document 1, Patent Document 2 and Patent Document 3), it has not been reported concretely that a novel 2,6-diaminopyrimidine derivative which has 6-amino moiety of the present invention, and also it has not been disclosed that a novel 2,6-diaminopyrimidine derivative of the present invention has an excellent BTK inhibitory effect.

PRIOR ART DOCUMENTS

PATENT DOCUMENTS

[Patent Document 1] WO 2008/033834
[Patent Document 2] WO 2009/137596
[Patent Document 3] WO 2013/083666

NON-PATENT DOCUMENTS

[Non-Patent Document 1] Satterthwaite, A. B. and Witte, O. N., Immunol. Rev., 2000, 175, 120-127
[Non-Patent Document 2] Kurosaki T., Curr. Opin. Immunol., 2000, 12, 276-281
[Non-Patent Document 3] Davis R. E. et al., Nature, 2010, 463, 88-92
[Non-Patent Document 4] Ellmeier W. et al., FEBS J., 2011, 278, 1990-2000
[Non-Patent Document 5] Halcomb K. E., Mol. Immunol., 2008, 46(2), 233-241
[Non-Patent Document 6] Jansson L. and Holmdahl R. , Clin. Exp. Immunol., 1993, 94, 459-465

SUMMARY OF INVENTION

PROBLEMS TO BE SOLVED BY THE INVENTION

An object of the present invention is to provide a pharmaceutical, particularly a novel 2,6-diaminopyrimidine derivative having a BTK inhibitory effect, or a pharmaceutically acceptable salt thereof.

MEANS OF SOLVING THE PROBLEMS

The present invention is achieved by the following (1) and (2) :

(1) A 2, 6-diaminopyrimidine derivative represented by the following formula (I):

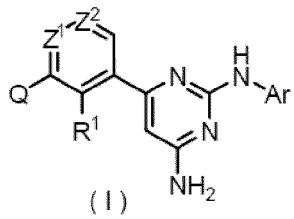

(I)

wherein

$R^1$ represents a substituted or unsubstituted lower alkyl group, or a substituted or unsubstituted alkoxy group,
Ar represents a substituted or unsubstituted aryl group, or a substituted or unsubstituted heteroaryl group,
$Z^1$ and $Z^2$ represent carbon atoms, or either 1 or 2 of the $Z^1$ and $Z^2$ represent nitrogen atoms,
Q is selected from a structure (a) and (b) described below:

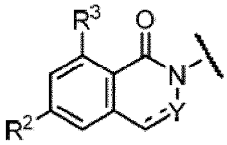

(a) ,

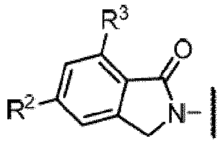

(b)

$R^2$ represents a substituted or unsubstituted lower alkyl group, or
a substituted or unsubstituted cycloalkyl group,
$R^3$ represents a hydrogen atom or a halogen atom,
Y represents a nitrogen atom or a carbon atom, and the bond drawn with a dotted line parallel to a solid line on structure (a) represents either double bond or single bond, or a pharmaceutically acceptable salt thereof;

(2) The 2,6-diaminopyrimidine derivative according to (1), wherein Q is a structure (a), or a pharmaceutically acceptable salt thereof.

EFFECT OF THE INVENTION

The present inventors have intensively studied so as to achieve the above object and found that a novel 2,6-diaminopyrimidine derivative represented by formula (I) shown above and a pharmaceutically acceptable salt thereof have an excellent BTK inhibitory effect, pharmacokinetics and thus completed the present invention.
The compound provided by the present invention is useful as a preventive or therapeutic medicine (pharmaceutical composition) for diseases which are known to be involved in abnormal cell response through BTK, for example, self-immune diseases, inflammatory diseases, bone diseases, and cancers such as lymphoma. The compound is also useful, as a BTK inhibitor, for reagents to be used in tests and researches.

BRIEF DESCRIPTION OF THE DRAWINGS

Figure 1 shows that the compounds of Example 1 and 2 inhibit the BCR signal in the Ramos cells in a concentration dependent manner and inhibit the flux of calcium into the cells (Test Example 3).

DESCRIPTION OF EMBODIMENTS

The present invention will be described in detail below.

A novel 2,6-diaminopyrimidine derivative of the present invention is a compound represented by formula (I) shown below:

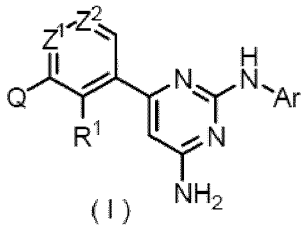

(I)

wherein

$R^1$ represents a substituted or unsubstituted lower alkyl group, or a substituted or unsubstituted alkoxy group,
Ar represents a substituted or unsubstituted aryl group, or a substituted or unsubstituted heteroaryl group,
$Z^1$ and $Z^2$ represent carbon atoms, or either 1 or 2 of the $Z^1$ and $Z^2$ represent nitrogen atoms,
Q is selected from a structure (a) and (b) described below:

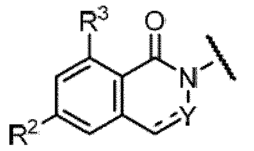

(a)

,

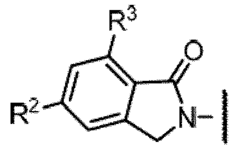

(b)

wherein $R^2$ represents a substituted or unsubstituted lower alkyl group, or a substituted or unsubstituted cycloalkyl group,
$R^3$ represents a hydrogen atom or a halogen atom,
Y represents a nitrogen atom or a carbon atom, and the bond drawn with a dotted line parallel to a solid line on structure (a) represents either double bond or single bond.

In formula (I) shown above, examples of the halogen atom include fluorine, chlorine, and bromine.

An aryl group moiety of the substituted or unsubstituted aryl group may be any of aryl groups having 6 to 14 carbon atoms, and specific examples thereof include phenyl, naphthyl, and indenyl, etc.

The heteroaryl moiety of the substituted or unsubstituted heteroaryl group may be any of heteroaryl groups including, for example, monocyclic aromatic heterocyclic ring group or fused aromatic heterocyclic ring group. The monocyclic aromatic heterocyclic ring group includes, for example, 5- or 6-membered monocyclic aromatic heterocyclic ring group having at least one heteroatom selected from a nitrogen atom, a sulfur atom, and an oxygen atom. Specific examples thereof include pyrrolyl, imidazolyl, pyrazolyl, thienyl, thiazolyl, furanyl, pyridyl, pyrimidinyl and pyridazyl. The fuse aromatic heterocyclic ring includes, for example, a fused bicyclic heterocyclic group in which 3- to 8-membered rings are condensed, and further including at least one heteroatom selected from a nitrogen atom, a sulfur atom, and an oxygen atom. Specific examples thereof include tetrahydroisoquinolyl, benzothiophenyl, benzimidazolyl, benzooxazolyl, benzothiazolyl, indolyl, and isoquinolyl etc.

An alkyl group moiety of 'the substituted or unsubstituted lower alkyl group' and 'the substituted or unsubstituted cycloalkyl group' may be any of linear, branched and cyclic alkyl groups having 1 to 3 carbon atoms, and specific examples thereof include a methyl group, an isopropyl group, a cyclopropyl group, and a tert-butyl group etc.

An alkoxy group moiety of the substituted or unsubstituted alkoxy group may be any of linear, branched, or cyclic alkyl group having 1 to 3 carbon atoms, and specific examples thereof include a methoxy group, an ethoxy group, an isopropyloxy group, and a cyclopropyloxy group etc.

As a substituent of the substituted or unsubstituted aryl group, the substituted or unsubstituted heteroaryl group, the substituted or unsubstituted lower alkyl group, or the substituted or unsubstituted alkoxy group, one, or two or more of any kind of substituent (s) may be attached at any chemically possible position. When the above group have two or more substituents, these substituents may be the same or different, and examples of the substituent include a halogen atom, a substituted or unsubstituted alkyl group, a substituted or unsubstituted alkoxy group, a substituted or unsubstituted amino group, a nitro group, a cyano group, a hydroxyl group, a substituted or unsubstituted alkylamino group, a substituted or unsubstituted carbamoyl group, a carboxyl group, a formyl group, an acetyl group, a benzoyl group, and a substituted

or unsubstituted acylamino group.

Also these substituents may be combined each other to form a saturated or unsaturated ring.

Isomers may sometimes exist in the compound (I) of the present invention, depending on the kind of the substituent. In the present description, the isomers may be sometimes described by a chemical structure of only one form thereof. The present invention includes all isomers (geometrical isomer, optical isomer, tautomer, etc.) which can be structurally formed, and also includes isomers alone, or a mixture thereof.

The specific examples of the compound (I) of the present invention and a pharmaceutically acceptable salt thereof are the following compounds:

2-(3-{6-Amino-2-[(4-morpholinophenyl)amino]pyrimidin-4-yl}-2-( hydroxymethyl)phenyl)-6-cyclopropyl-8-fluoroisoquinolin-1(2H)-one

2-(3-{6-Amino-2-[(1-methyl-1*H*-pyrazol-4-yl)amino]pyrimidin-4-y 1}-2-(hydroxymethyl)phenyl)-6-cyclopropyl-8-fluoroisoquinolin-1(2*H*)-one

2-(3-{6-Amino-2-[(1-methyl-1*H*-pyrazol-4-yl)amino]pyrimidin-4-y l}-2-(hydroxymethyl)phenyl)-6-cyclopropyl-8-fluoro-3,4-dihydro isoquinolin-1(2*H*)-one

2-(3-{6-Amino-2-[(4-morpholinophenyl)amino]pyrimidin-4-yl}-2-( hydroxymethyl)phenyl)-6-cyclopropyl-8-fluoro-3,4-dihydroisoqui nolin-1(2*H*)-one

4-({4-Amino-6-[3-(6-cyclopropyl-8-fluoro-1-oxoisoquinolin-2(1*H* )-yl)-2-(hydroxymethyl)phenyl]pyrimidin-2-yl}amino)-1-methyl-1 H-pyrrole-2-carbonitrile

2-(3-{6-Amino-2-[(4-methoxyphenyl)amino]pyrimidin-4-yl}-2-(hyd roxymethyl)phenyl)-6-cyclopropyl-8-fluoroisoquinolin-1(2*H*)-one

2-(3-{6-Amino-2-[(1-methyl-1*H*-pyrazol-4-yl)amino]pyrimidin-4-y 1}-2-(hydroxymethyl)phenyl)-6-(tert-butyl)-8-fluoroisoquinolin -1(2H)-one

2-[3-(6-Amino-2-{[1-(cyclopropylmethyl)-1*H*-pyrazol-4-yl]amino} pyrimidin-4-yl)-2-(hydroxymethyl)phenyl]-6-cyclopropyl-8-fluor oisoquinolin-1(2*H*)-one

2-(3-{6-Amino-2-[(1-cyclopropyl-1*H*-pyrazol-4-yl)amino]pyrimidi n-4-yl}-2-(hydroxymethyl)phenyl)-6-cyclopropyl-8-fluoroisoquin olin-1(2*H*)-one

4-({4-Amino-6-[3-(6-cyclopropyl-8-fluoro-1-oxoisoquinolin-2(1*H* )-yl)-2-(hydroxymethyl)phenyl]pyrimidin-2-yl}amino)-1-cyclopro pyl-1H-pyrrole-2-carbonitrile

2-{3-[6-Amino-2-(pyridin-2-ylamino)pyrimidin-4-yl]-2-(hydroxym ethyl)phenyl}-6-cyclopropyl-8-fluoroisoquinolin-1(2*H*)-one

2-(3-{6-Amino-2-[(1-methyl-1*H*-pyrazol-3-yl)amino]pyrimidin-4-y 1}-2-(hydroxymethyl)phenyl)-6-cyclopropyl-8-fluoroisoquinolin-1(2*H*)-one

2-[3-(6-Amino-2-{[1-(2,2-difluoroethyl)-1*H*-pyrazol-4-yl]amino} pyrimidin-4-yl)-2-(hydroxymethyl)phenyl]-6-cyclopropyl-8-fluor oisoquinolin-1(2*H*)-one

2-(3-{6-Amino-2-[(1-isopropyl-1*H*-pyrazol-4-yl)amino]pyrimidin-4-yl}-2-(hydroxymethyl)phenyl)-6-cyclopropyl-8-fluoroisoquinol in-1(2*H*)-one

2-[3-(6-Amino-2-{[4-(4-methylpiperazin-1-yl)phenyl]amino}pyrim idin-4-yl)-2-(hydroxymethyl)phenyl]-6-cyclopropyl-8-fluoroisoq uinolin-1(2*H*)-one

2-[3-(6-Amino-2-{[4-(morpholinomethyl)phenyl]amino}pyrimidin-4 -yl)-2-(hydroxymethyl)phenyl]-6-cyclopropyl-8-fluoroisoquinoli n-1(2*H*)-one

2-(3-{2-[(5-Acetyl-1-methyl-1*H*-pyrrol-3-yl)amino]-6-aminopyrim idin-4-yl}-2-(hydroxymethyl)phenyl)-6-cyclopropyl-8-fluoroisoq uinolin-1(2*H*)-one

2-(3-{2-[(1*H*-Pyrazol-4-yl)amino]-6-aminopyrimidin-4-yl}-2-(hyd roxymethyl)phenyl)-6-cyclopropyl-8-fluoroisoquinolin-1(2*H*)-one

2-(3-{6-Amino-2-[(1-methyl-1*H*-pyrrol-3-yl)amino]pyrimidin-4-yl }-2-(hydroxymethyl)phenyl)-6-cyclopropyl-8-fluoroisoquinolin-1 (2*H*)-one

2-(3-{6-Amino-2-[(1-methyl-1*H*-pyrazol-4-yl)amino]pyrimidin-4-y 1}-2-(hydroxymethyl)phenyl)-6-cyclopropyl-8-fluorophthalazin-1 (2*H*)-one

2-(3-{6-Amino-2-[(1-cyclopropyl-1*H*-pyrazol-4-yl)amino]pyrimidi n-4-yl}-2-(hydroxymethyl)phenyl)-6-cyclopropyl-8-fluorophthala zin-1(2*H*)-one

2-(4-{6-Amino-2-[(1-methyl-1*H*-pyrazol-4-yl)amino]pyrimidin-4-y 1}-3-(hydroxymethyl)pyridin-2-yl)-6-cyclopropyl-8-fluoroisoqui nolin-1(2*H*)-one

2-(4-{6-Amino-2-[(1-cyclopropyl-1*H*-pyrazol-4-yl)amino]pyrimidi n-4-yl}-3-(hydroxymethyl)pyridin-2-yl)-6-cyclopropyl-8-fluoroi soquinolin-1(2*H*)-one

4-({4-Amino-6-[3-(6-cyclopropyl-8-fluoro-1-oxophthalazin-2(1*H*) -yl)-2-(hydroxymethyl)phenyl]pyrimidin-2-yl}amino)-1-methyl-1*H* -pyrrole-2-carbonitrile

2-(3-{6-Amino-2-[(1-methyl-1*H*-pyrazol-4-yl)amino]pyrimidin-4-y 1}-2-(hydroxymethyl)phenyl)-8-fluoro-6-(1-methylcyclopropyl)is oquinolin-1(2*H*)-one

2-{3-[6-Amino-2-({1-[1-(hydroxymethyl)cyclopropyl]-1H-pyrazol-4-yl}amino)pyrimidin-4-yl]-2-(hydroxymethyl)phenyl}-6-cyclopro pyl-8-fluoroisoquinolin-1(2H)-one

Examples of the pharmaceutically acceptable salt of the compound (I) of the present invention include inorganic acid salts with hydrochloric acid, sulfuric acid, carbonic acid, and phosphoric acid; and organic acid salts with fumaric acid, maleic acid, methanesulfonic acid, and p-toluenesulfonic acid, etc. The present invention also includes ammonium salts, in addition to alkali metal salts with sodium and potassium; alkaline earth metal salts with magnesium and calcium; organic amine salts with lower alkylamine and lower alcoholamine; and basic amino acid salts with lysine, arginine, and ornithine.

The compound (I) of the present invention and a pharmaceutically acceptable salt thereof can be produced, for example, by methods shown below. When defined groups vary under the conditions of an implemental method in the production method shown below, or are unsuited to carry out the method, it is possible to easily produce them by a method which is usually used in organic synthetic chemistry, for example, a method of applying means such as protection or deprotection of a functional group [T. W. Greene, Protective Groups in Organic Synthesis 3rd Edition, John Wiley&Sons, Inc., 1999]. If necessary, the order of a reaction step such as introduction of substituents can also be changed.

Meanings of abbreviations and symbols used in the following description are as follows.

DCM: dichloromethane
DCC: *N,N'*-dicyclohexylcarbodiimide
EDC: 1-ethyl-3-(3-dimethylaminopropyl)carbodiimide hydrochloride
HOBt: 1-hydroxybenzotriazole
THF: tetrahydrofuran
DIEA: N,N-diisopropylethylamine
DMF: N,N-dimethylformamide
DMSO: dimethyl sulfoxide
TEA: triethylamine
$CDCl_3$: deuterated chloroform

[Method for Production of the Compound (I) of the Present Invention]

The compound of the present invention represented by formula (I) can be produced, for example, according to Scheme 1:

[Scheme 1]

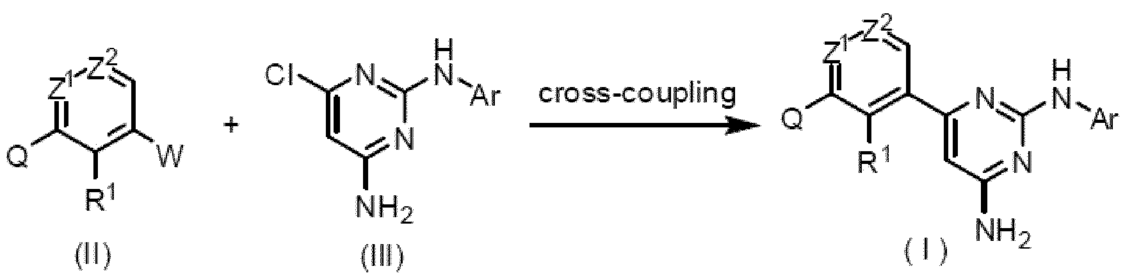


(II) (III) (I)

wherein $R^1$, Q, Ar, $Z^1$ and $Z^2$ are as defined above, and W represents a boronyl group or a boronic acid ester group.

The compound (I) of the present invention can be produced by a cross-coupling reaction such as Suzuki coupling reaction, using a compound (II) and a compound (III) (see, for example, known literatures (N. Miyaura et al., J. Am. Chem. Soc., 107, 972 (1985)., N. Miyaura, A. Suzuki, Chem. Rev. 95, 2457 (1995) with respect to the conditions of the Suzuki coupling reaction)). That is, the reaction can be carried out in the presence of a metal catalyst such as palladium or nickel using a base and additives, if necessary. Examples of a solvent used in the reaction include THF, dioxane, toluene, dimethoxyethane, methanol, ethanol, and acetonitrile, etc. It is also suitable to use two or more kinds of these solvents, or to use them in combination with water. The solvent is preferably a mixed solvent of THF and water, or a mixed solvent of toluene, methanol and water, or dioxane. The compound (II) is preferably used in an equivalent or

excess amount, and more preferably in an amount of from 1 equivalent to 10 equivalents, based on the compound (III). If necessary, a base may be added so as to accelerate the reaction, and sodium carbonate, cesium carbonate, and potassium carbonate are usually used as the base. The amount of the base to be used is from 1 equivalent to 10 equivalents, and preferably from 1 equivalent to 5 equivalents, based on the compound (III). It is possible to use, as a metal catalyst, a commercially available palladium catalyst (for example, $PdCl_2(dppf)$, $Pd_2(dba)_3$, $Pd(PPh_3)_4$, etc.) which is used in the cross-coupling, and the catalyst is preferably used in a catalytic amount, that is, an amount of from 0.1 equivalent to 0.5 equivalent based on the compound (III).

If necessary, additives can be added so as to accelerate the reaction. The additive includes, for example, rac-BINAP and can be used in the amount of from 0.01 equivalent to 1 equivalent based on the compound (III). The product can be synthesized by the reaction at a temperature ranging from 0°C to 200°C for several minutes to several days, preferably from 10°C to 100°C for 1 hour to 36 hours. It is also possible to synthesize the product by reacting under the temperature condition of from 60°C to 150°C for several minutes to several hours, using a microwave synthesis equipment.

The compound (II) used as a starting material of Scheme 1 can be produced, for example, by the method shown in Scheme 2:

[Scheme 2]

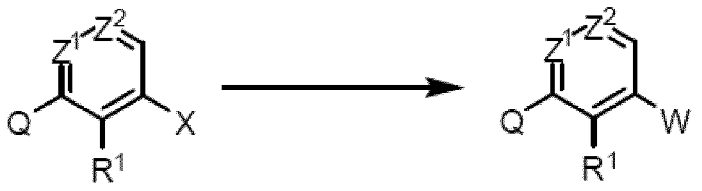

(IV) (II)

wherein $R^1$, Q, $Z^1$, $Z^2$ and W are as defined above, and X represents a halogen atom.

The compound (II) can be produced by activating the compound (IV) with *n*-butyllithium, and then reacting the activated compound with a boric acid ester. That is, the compound (II) can be obtained by lithiation of the compound (IV) with 1 to 5 molar equivalents, preferably 1 to 1.5 molar equivalents of *n*-butyllithium, and reacting the lithiated compound with 1 to 5 molar equivalents, preferably 1 to 1.5 molar equivalents of a boric acid ester.

The solvent may be any solvent as long as it is inert to the reaction and is not particularly limited, and THF can be preferably used.

The reaction temperature is usually from -100°C to -30°C, and preferably from -80°C to -60°C. The reaction time is not particularly limited, and is usually from 0.1 hour to 12 hours, and the reaction time of from 0.2 hour to 6 hours is exemplified as a preferable example.

The compound (II) can also be obtained by reacting the compound (IV) with 1 to 5 molar equivalents, preferably 1 to 1.5 molar equivalents of metallic magnesium and a catalytic amount of iodine in an ether-based solvent at a temperature of from -10°C to a boiling point of the solvent to be used to obtain a Grignard reagent, and then reacting the Grignard reagent with 1 to 5 molar equivalents, preferably 1 to 1.5 molar equivalents of a boric acid ester. The reaction temperature is usually from -30°C to -100°C, and preferably from -60°C to -80°C. The reaction time is not particularly limited and is usually from 0.1 hour to 12 hours, and the reaction time of from 0.2 hour to 6 hours is exemplified as a preferable example.

Furthermore, the compound (II) can be obtained by subjecting the compound (IV) and 1 to 5 molar equivalents, preferably 1 to 3 molar equivalents of a diboron ester to a coupling reaction in the presence of a metal catalyst such as palladium and nickel and a base in an organic solvent.

It is possible to use, as the metal catalyst, a commercially available palladium catalyst (for example, $PdCl_2(dppf)$, $Pd_2(dba)_3$, $Pd(PPh_3)_4$, etc.) which is used in the cross-coupling, and the catalyst is preferably used in a catalytic amount, that is, an amount of from 0.1 equivalent to 0.5 equivalent based on the compound (IV) to be used in the cross-coupling. Potassium acetate is usually used as the base. The amount of the base to be used is from 1 equivalent to 10 equivalents based on the compound (IV), preferably from 1 equivalent to 5 equivalents, based on the compound (IV).

The solvent may be any solvent as long as it is inert to the reaction and is not particularly limited, and dioxane can be preferably used.

The reaction temperature is usually from 0°C to 200°C, preferably from 10°C to 100°C. The reaction time is not particularly limited and the reaction time of from 0.2 hour to 48 hours is usually exemplified, and the reaction time of from 1 hour to 36 hours is exemplified as a preferable example.

It is desired that any of these reactions are carried out in an inert gas (argon, nitrogen etc.) atmosphere, under anhydrous conditions.

The compound (IV) to be used as a starting material of Scheme 2 can be produced, for example, by the method shown in Scheme 3:

[Scheme 3]

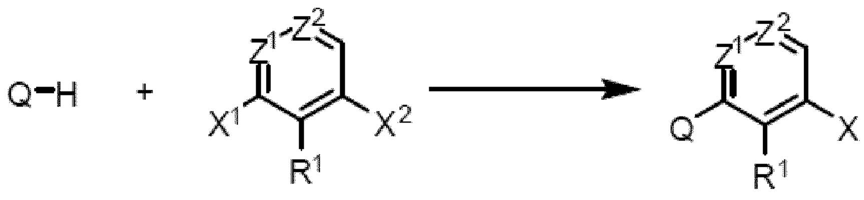

(V) (VI) (IV)

wherein $R^1$, Q, $Z^1$, $Z^2$ and X are as defined above, and $X^1$ and $X^2$ of the compound (VI) represent the same or different halogen atoms.

The compound (IV) can be obtained by reacting compound (V) with 1 to 5 molar equivalents, preferably 1.5 to 3 molar equivalents of compound (VI) in a polar solvent in the presence of metal catalyst and base.

The solvent may be any solvent as long as it is inert to the reaction and is not particularly limited, and dioxane can be preferably used.

In carrying out the coupling reaction, the compound (IV) can also be produced by optionally protecting or deprotecting an $R^1$ group of the compound (VI), appropriately combining methods to be usually used in organic synthetic chemistry. For example, it is possible to use protection or deprotection of a functional group, such as hydroxyl or amino group of the compound (VI) [T. W. Greene, Protective Groups in Organic Synthesis 3rd Edition, John Wiley&Sons, Inc., 1999] and aldehyde derivative which is hydroxyl group precursor of the compound (VI).

The reaction can be carried out at a temperature of from 80°C to 200°C for 0.5 hour to 200 hours, preferably from 100°C to 150°C for 1 hour to 100 hours. It is also possible to perform the reaction using microwave synthesis equipment.

It is possible to use, as the metal catalyst, a commercially available palladium catalyst (for example, $PdCl_2$(dppf), $Pd_2(dba)_3$, $Pd(PPh_3)_4$, etc.) or copper(I) iodide which is used in the coupling reaction, and the catalyst is preferably used in a catalytic amount, that is, an amount of from 0.01 equivalent to 2 equivalents based on the compound (V) to be used in the coupling.

Examples of the base to be used include potassium carbonate, sodium carbonate, cesium carbonate and sodium hydrogen carbonate, and cesium carbonate and sodium hydrogen carbonate can be preferably used. The amount of the base to be used is from 1 molar equivalent to 10 molar equivalents based on the compound (V), preferably from 2 molar equivalents to 5 molar equivalents, based on the compound (V). And if necessary, xantphos can be used as additive to the reaction in the amount of 0.1 equivalent to 0.5 equivalent based on the compound (V).

The compound (VI) can be obtained as a commercially available product, or can be obtained by a well-known procedure or the procedure according to it.

Among the compounds (V), which are used as starting materials in Scheme 3, the compound (V-a-i) and (V-a-ii), in which Q is structure (a) and a bond drawn with a dotted line parallel to a solid line is a double bond, can be produced, for example, by the method shown in Scheme 4:

[Scheme 4]

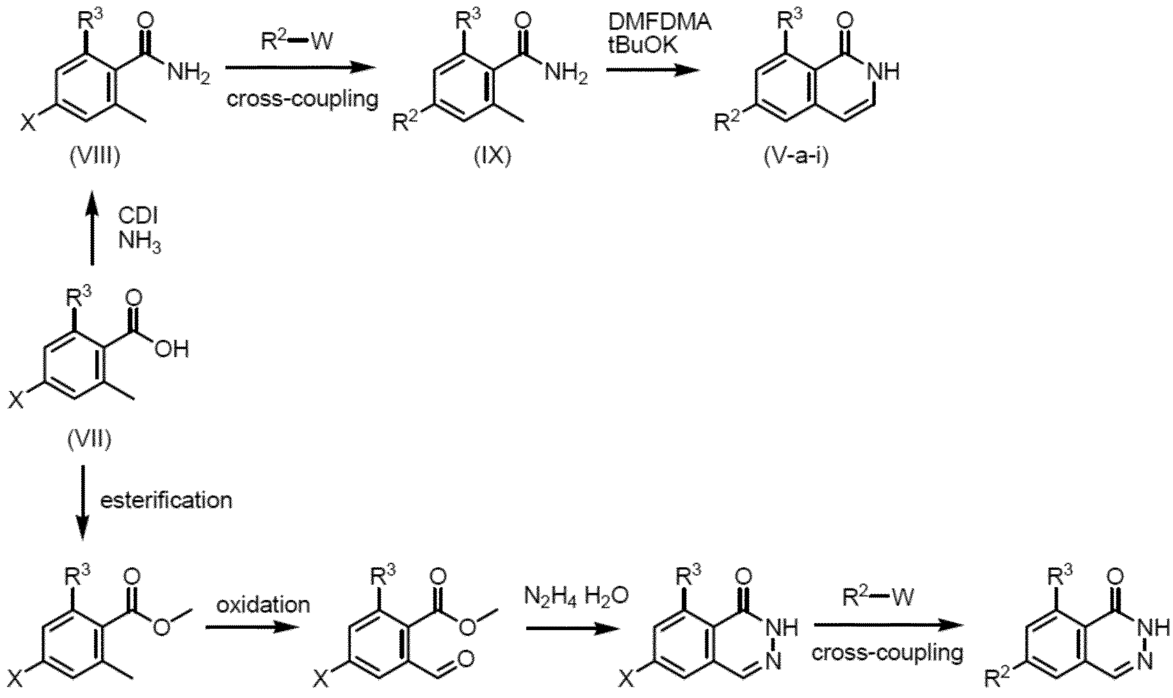


(VIII) (IX) (V-a-i) (VII) (X) (XI) (XII) (V-a-ii)

wherein X, W, $R^2$ and $R^3$ are as defined above.

The compound (V-a-i) can be produced by a cyclization reaction of the compound, which is obtained by a cross-coupling reaction for introducing $R^2$ group after converting the carboxylic acid group of the compound (VII) to carbamoyl group, with *N,N*-dimethylformamide dimethyl acetal.

On the other hand, the compound (V-a-ii) can be produced by introducing $R^2$ group by a cross-coupling reaction of the compound, which is obtained by a cyclization reaction with hydrazine after converting the carboxylic acid group of the compound (VII) to ester group then oxidizing of the methyl group of the benzene ring to aldehyde to afford the compound (XI).

The compound (VII), (X) and $R^2$-W to be used as a starting material of Scheme 4 can be obtained as a commercially available product, or can be obtained by a well-known procedure or the procedure according to it.

Among the compounds (V), which are used as starting materials in Scheme 3, the compound (V-a-iii), in which Q is structure (a) and a bond drawn with a dotted line parallel to a solid line is a single bond, can be produced, for example, by the method shown in Scheme 5:

[Scheme 5]

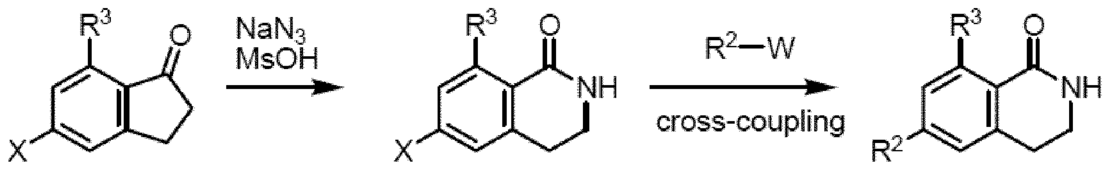


(XV) (XVI) (V-a-iii)

wherein X, W, $R^2$ and $R^3$ are as defined above.

The compound (V-a-iii) can be produced by a cross-coupling reaction of the compound, which is obtained by a Schmidt rearrangement reaction of the compound (XV) with sodium azide, for introducing $R^2$ group.

The compound (XV) and $R^2$-W to be used as a starting material of Scheme 5 can be obtained as a commercially available product, or can be obtained by a well-known procedure or the procedure according to it.

Among the compounds (V), which are used as starting materials in Scheme 3, the compound (V-b), in which Q is structure (b), can be produced, for example, by the method shown in Scheme 6:

[Scheme 6]

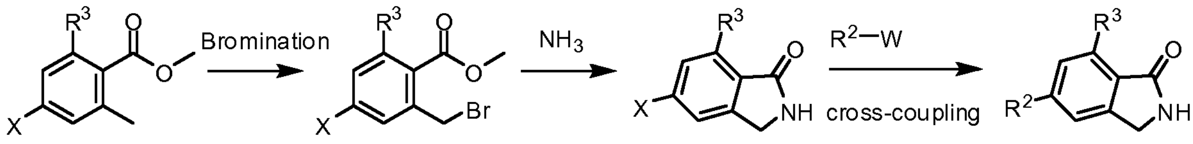


(X) (XIII) (XIV) (V-b)

wherein X, W, $R^2$ and $R^3$ are as defined above.

The compound (V-b) can be produced by bromination of the methyl group on the benzene ring of the compound (X), subsequent cyclization reaction of the compound (XIII) by ammonia and then a cross-coupling reaction of the compound (XIV) with boronic acid $R^2$-W.

The compound (X) and $R^2$-W to be used as a starting material of Scheme 6 can be obtained as a commercially available product, or can be obtained by a well-known procedure or the procedure according to it.

The compound (III) to be used as a starting material of Scheme 1 can be produced, for example, by the method shown in Scheme 7:

[Scheme 7]

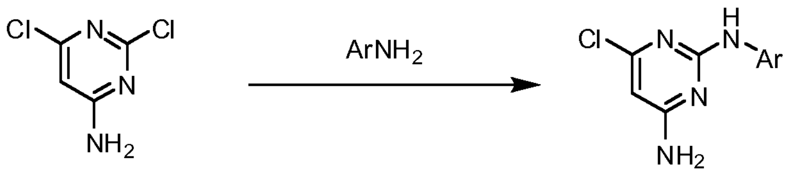


(III)

wherein Ar are as defined above.

The compound (III) can be obtained by reacting $ArNH_2$ with 1 to 5 molar equivalents, preferably 1 to 1.5 molar equivalents of 4-amino-2,6-dichloropyrimidine in a polar solvent and, if necessary, in the presence of an acid catalyst.

The solvent may be any solvent as long as it is inert to the reaction and is not particularly limited, and dimethoxyethane and ethanol can be preferably used.

The reaction temperature is usually from 0°C to 200°C, preferably from 50°C to 150°C. The reaction time is not particularly limited and the reaction time of from 0.2 hour to 24 hours is usually exemplified, and the reaction time of from 1 hour to 18 hours is exemplified as a preferable examples.

4-Amino-2,6-dichloropyrimidine to be used as a starting material of Scheme 7 can be obtained as a commercially available product, and $ArNH_2$ can be obtained as a commercially available product, or can be obtained by a well-known procedure or the procedure according to it.

The compound of the present invention represented by formula (I) can be produced, for example, according to Scheme 8:

[Scheme 8]

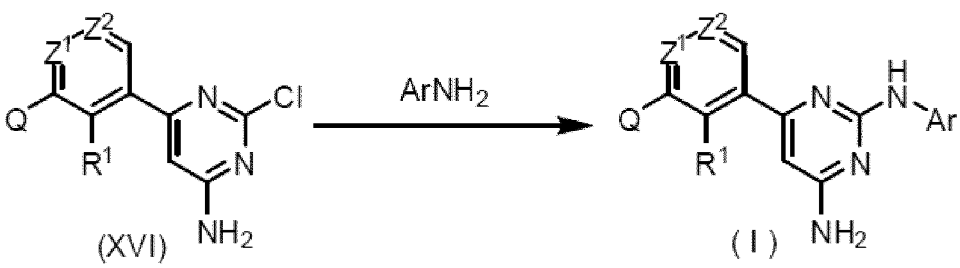


(XVI) (I)

wherein $R^1$, Q, Ar, $Z^1$ and $Z^2$ are as defined above.

The compound (I) of the present invention can be produced by a substitution reaction of the compound (XVI) with $ArNH_2$. The reaction conditions are the same as described in the Scheme 7, which is mentioned on the production of the compound (III).

The compound (XVI) to be used as a starting material of Scheme 8 can be produced, for example, by the method shown in Scheme 9:

[Scheme 9]

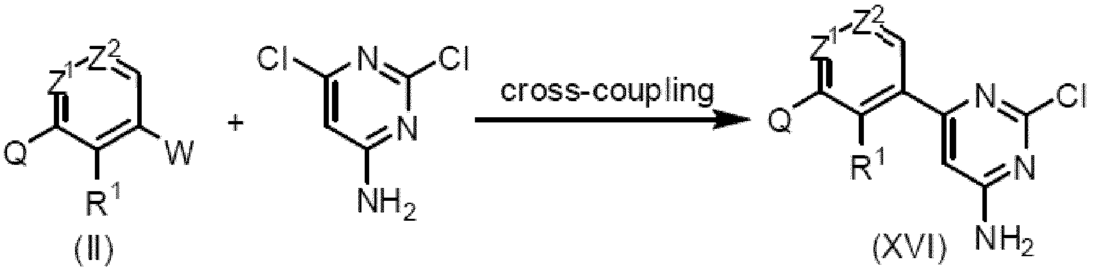


wherein $R^1$, Q, W, $Z^1$ and $Z^2$ are as defined above.

The compound (XVI) can be produced by a cross-coupling reaction of the compound (II) and 4-amino-2,6-dichloropyrimidine. The reaction conditions are the same as described in the Scheme 1, which is mentioned on the production of the compound (I) of the present invention.

In the scheme shown above, a boronyl group represented by W may be in the form of a salt of alkali metal or alkaline earth metal, and specific examples of the boronic acid ester group include boronic acid ester groups such as a boronic acid dimethyl ester group, a boronic acid diethyl ester group, a boronic acid dibutyl ester group, a boronic acid dicyclohexyl group, a boronic acid ethylene glycol ester group, a boronic acid propylene glycol ester group (a boronic acid 1,2-propanediol ester group, a boronic acid 1, 3-propanediol ester group), a boronic acid neopentyl glycol ester group, a boronic acid catechol ester group, a boronic acid glycerin ester group, a boronic acid trimethylolethane ester group, a boronic acid diethanolamine ester group, and a boronic acid triethanolamine ester group; and boronic acid anhydride groups.

It is possible to obtain the compound (I) having the desired functional group at the desired position of the present invention by appropriately using the above methods in combination, and then carrying out a method usually used in organic synthetic chemistry (for example, an alkylation reaction of an amino group, an oxidizing reaction of alkylthio group into a sulfoxide group or a sulfone group, a reaction of converting an alkoxy group into a hydroxyl group, or a reaction of inversely converting the group).

[Applications of Compound (I) of the Present Invention]

The compound (I) or a pharmaceutically acceptable salt thereof of the present invention can be prepared into a form of a conventional pharmaceutical formulation (pharmaceutical composition), which is suited for oral administration, parenteral administration, or local administration.

Formulations for oral administration include solid formulations such as tablets, granules, powders, and capsules; and liquid formulations such as syrups. These formulations can be prepared by a conventional method. The solid formulations can be prepared by using conventional pharmaceutical carriers, for example, starches such as lactose and corn starch; crystalline celluloses such as microcrystalline cellulose; and hydroxypropyl cellulose, calcium carboxymethyl cellulose, talc, and magnesium stearate. Capsules can be prepared by encasing thus prepared granules or powders in capsules. Syrups can be prepared by dissolving or suspending the compound (I) or a pharmaceutically acceptable salt thereof of the present invention in an aqueous solution containing sucrose and carboxymethyl cellulose.

Formulations for parenteral administration include injections such as instillation. Injection formulations can also be prepared by a conventional method, and can be appropriately incorporated into isotonic agents (for example, mannitol, sodium chloride, glucose, sorbitol, glycerol, xylitol, fructose, maltose, mannose), stabilizers (for example, sodium sulfite, albumin), and antiseptics (for example, benzyl alcohol, methyl p-oxybenzoate).

The dosage of the compound (I) or a pharmaceutically acceptable salt thereof of the present invention can vary depending on severity of disease, age and body weight of the patient, and dosage form, and is usually within a range from 1 mg to 1,000 mg per day for adults. The compound or a pharmaceutically acceptable salt thereof can be administered

once, or dividedly administered twice or three times according to an oral or parenteral route.

The compound (I) or a pharmaceutically acceptable salt thereof of the present invention can also be used, as a BTK inhibitor, for reagents to be used in tests and researches.

## EXAMPLES

The present invention will be more specifically described below by way of Examples and Test Examples, but the present invention is not limited to these Examples.

Identification of the compound was carried out by hydrogen nuclear magnetic resonance spectrum ($^1$H-NMR) and mass spectrum (MS). $^1$H-NMR is measured at 400 MHz, unless otherwise specified, and exchangeable hydrogen cannot be sometimes clearly observed depending on the compound and measurement conditions. br. means a broad signal (broad).

HPLC preparative chromatography was carried out by a commercially available ODS column in a gradient mode using water/methanol (containing formic acid) as eluents, unless otherwise specified.

### Example 1

2-(3-{6-Amino-2-[(4-morpholinophenyl)amino]pyrimidin-4-yl}-2-( hydroxymethyl)phenyl)-6-cyclopropyl-8-fluoroisoquinolin-1(2H)-one

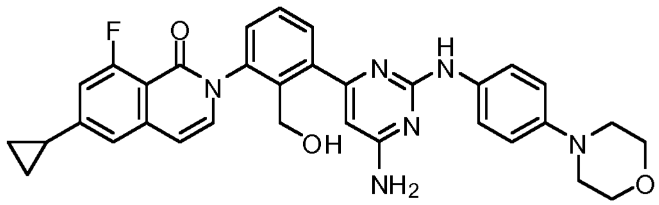

(First Step)

Under nitrogen atmosphere, 4-bromo-2-fluoro-6-methylbenzoic acid (13.0 g, 55.8 mmol) was dissolved in THF (100 mL). To this solution, 1,1'-carbonyldiimidazole (11.8 g, 72.5 mmol) was added at 0°C then stirred at 0°C for 2 h. To this reaction mixture, 28% ammonia solution (10 mL) was added dropwise during a period of 5 min and then stirred at ambient temperature for further 2 days. The reaction mixture was concentrated to around 50 mL under reduced pressure, and 6 M hydrochloric acid solution (30 mL) was added, and then extracted with ethyl acetate (2x100 mL). The combined organic layer was washed with saturated sodium hydrogen carbonate solution and brine, dried over sodium sulfate, filtered and concentrated to afford 4-bromo-2-fluoro-6-methylbenzamide (11.0 g).

$^1$H NMR (400 MHz, $CDCl_3$) δ7.22 (dt, J = 1.8, 0.8 Hz, 1H), 7.15 (dd, J = 9.0, 1.9 Hz, 1H), 6. 06 - 5.60 (m, 2H), 2.44 (s, 3H) ; LCMS (m/z) : 231.9 $[M+H]^+$.

(Second Step)

To a mixed solution of 4-bromo-2-fluoro-6-methylbenzamide (11.0 g) in toluene (110 mL) and water (11 mL), cyclopropylboronic acid (6.11 g, 71.1 mmol), tricyclohexylphosphine (0.80 g, 2.84 mmol), tris(dibenzylideneacetone)dipalladium (0) (0.43 g, 0.47 mmol) and potassium carbonate (19. 65 g, 142.0 mmol) were added under nitrogen atmosphere and stirred at 115°C for 14 h. The mixture was cooled to ambient temperature, the precipitate was collected by filtration, washed with ether and water then dried to afford 4-cyclopropyl-2-fluoro-6-methylbenzamide (3.3 g). The filtrate was extracted with ethyl acetate (2x200 mL), and the combined organic layer was washed with brine, dried over sodium sulfate, filtered and concentrated. The crude material was purified by chromatography on silica gel, eluted with hexane/ethyl acetate to afford 4-cyclopropyl-2-fluoro-6-methylbenzamide (5.3 g).

$^1$H NMR (400 MHz, $CDCl_3$) δ6.80 - 6.70 (m, 1H), 6.60 (dd, J = 11.3, 1.6 Hz, 1H), 5.99 - 5.59 (m, 2H), 2.43 (s, 3H), 1.89 - 1.80 (m, 1H), 1.03 - 0.98 (m, 2H), 0.73 - 0.65 (m, 2H); LCMS (m/z): 194.0 $[M+H]^+$.

(Third Step)

To a solution of 4-cyclopropyl-2-fluoro-6-methylbenzamide (8.6 g, 44.5 mmol) similarly prepared according to

the procedure described in the Second Step in 2-methyltetrahydrofuran (100 mL), N,N-dimethylformamide dimethyl acetal (7.0 g, 58.8 mmol) was added under nitrogen atmosphere, and stirred at 60°C for 2 h. The reaction mixture was concentrated under reduced pressure, and 2-methyltetrahydrofuran (10 mL) was added to this crude material. To this solution, 1 mol/L potassium tert-butoxide in THF solution (68.1 mL, 68.1 mmol) was added dropwise, and stirred at 65°C for 1 day. The mixture was cooled to ambient temperature, and the reaction mixture was poured into 1 M hydrochloric acid solution (200 mL). To this solution, isopropyl alcohol (300 mL) was added and then the solvents were removed under reduced pressure. The precipitate was collected by filtration to afford 6-cyclopropyl-8-fluoroisoquinolin-1(2*H*)-one (7.7 g).

$^1$H NMR (400 MHz, DMSO-$d_6$) δ11.0 6 (s, 1H), 7.16 (d, J = 1.6 Hz, 1H), 7.11 (dd, J = 7.1, 5.7 Hz, 1H), 6.88 (dd, J = 13.3, 1.7 Hz, 1H), 6.41 (dd, J = 7.1, 2.3 Hz, 1H), 2.07 - 1.95 (m, 1H), 1.08 - 1.01 (m, 2H), 0.86 - 0.79 (m, 2H); LCMS (m/z): 204.1 [M+H] $^+$.

(Fourth Step)

To a solution of 6-cyclopropyl-8-fluoroisoquinolin-1(2*H*)-one (2.6 g, 12.8 mmol) in DMF (25 mL), 2-bromo-6-chlorobenzaldehyde (3.65 g, 16.63 mmol), potassium carbonate (3.54 g, 25. 6 mmol) and copper (I) iodide (0.49 g, 2.56 mmol) were added under nitrogen atmosphere and stirred at 110°C for 1 day. The reaction mixture was diluted with ethyl acetate (200 mL), filtered to remove insoluble material, and then the filtrate was washed with water and brine, dried over sodium sulfate, filtered and concentrated. The crude material was purified by chromatography on silica gel, eluted with hexane/ethyl acetate to afford 2-chloro-6-(6-cyclopropyl-8-fluoro-1-oxoisoquinolin-2(1*H*)-yl)b enzaldehyde (2.7 g).

$^1$H NMR (400 MHz, DMSO-$d_6$) δ10.18 (s, 1H), 7.84 - 7.78 (m, 1H), 7.75 (dd, J = 8.2, 1.3 Hz, 1H), 7.49 (dd, J = 7.8, 1.2 Hz, 1H), 7.41 (d, J = 7.5 Hz, 1H), 7.27 (d, J = 1.6 Hz, 1H), 7.00 (dd, J = 13.3, 1.6 Hz, 1H), 6.64 (dd, J = 7.5, 2.2 Hz, 1H), 2.14 - 2.01 (m, 1H), 1.14 - 1.06 (m, 2H), 0.92 - 0.83 (m, 2H); LCMS (m/z): 342.1 [M+H] +

(Fifth Step)

Under nitrogen atmosphere, a mixed solution of 2-chloro-6-(6-cyclopropyl-8-fluoro-1-oxoisoquinolin-2(1*H*)-yl)b enzaldehyde (2.5 g, 7.32 mmol) in DCM (26 mL) and isopropyl alcohol (13 mL) was cooled to 0°C. To this solution, sodium borohydride (0.42 g, 11.0 mmol) was added at 0°C and then stirred at 0°C for 2 h. Water (50 mL) was added to the reaction mixture, and extracted with ethyl acetate (2x50 mL). The combined organic layer was washed with water and brine, dried over sodium sulfate, filtered and concentrated to afford 2-[3-chloro-2-(hydroxymethyl)phenyl]-6-cyclopropyl-8-fluoroiso quinolin-1(2*H*)-one (2.3 g).

$^1$H NMR (400 MHz, $CDCl_3$) δ7.55 (dd, J = 8.1, 1.2 Hz, 1H), 7.40 (t, J = 8.0 Hz, 1H), 7.15 (dd, J = 8.0, 1.2 Hz, 1H), 7.08 - 7.00 (m, 2H), 6.82 (dd, J = 12.7, 1.7 Hz, 1H), 6.51 (dd, J = 7.4, 2.1 Hz, 1H), 4.71 - 4.61 (m, 1H), 4.46 (d, J = 11.9 Hz, 1H), 3.43 - 3.29 (m, 1H), 2.03 - 1.97 (m, 1H), 1.18 - 1.10 (m, 2H), 0.88 - 0.81 (m, 2H); LCMS (m/z): 343.9 [M+H] $^+$.

(Sixth Step)

To a solution of 2-[3-chloro-2-(hydroxymethyl)phenyl]-6-cyclopropyl-8-fluoroiso quinolin-1(2*H*)-one (2.26 g, 6.59 mmol) in DCM (30 mL), pyridine (2.36 mL, 29.3 mmol) and acetyl chloride (1.56 mL, 21.95 mmol) were added under nitrogen atmosphere, and stirred at ambient temperature for 1 day. Water (50 mL) was added to the reaction mixture, and extracted with ethyl acetate (2x50 mL). The combined organic layer was washed with water and brine, dried over sodium sulfate, filtered and concentrated. The crude material was purified by chromatography on silica gel, eluted with hexane/ethyl acetate to afford 2-chloro-6-(6-cyclopropyl-8-fluoro-1-oxoisoquinolin-2(1*H*)-yl)b enzyl acetate (2.3 g).

$^1$H NMR (400 MHz, $CDCl_3$) δ7.53 (dd, J = 8.2, 1.2 Hz, 1H), 7.46 - 7.39 (m, 1H), 7.22 (dd, J = 7.9, 1.3 Hz, 1H), 7.04 - 6.98 (m, 2H), 6.80 (dd, J = 12.6, 1.7 Hz, 1H), 6.43 (dd, J = 7.4, 2.1 Hz, 1H), 5.25 (d, J = 12.5 Hz, 1H), 4.98 (d, J = 12.4 Hz, 1H), 2.02 - 1.96 (m, 1H), 1.96 (s, 3H), 1.16 - 1.10 (m, 2H), 0.86 - 0.81 (m, 2H); LCMS (m/z): 386.0 [M+H] $^+$.

(Seventh Step)

To a solution of 2-chloro-6-(6-cyclopropyl-8-fluoro-1-oxoisoquinolin-2(1*H*)-yl)b enzyl acetate (4.8 g, 12.44 mmol) similarly prepared according to the procedure described in the Sixth Step in 1,4-dioxane (180 mL), bis(pinacolato)diboron (9.48 g, 37.3 mmol), bis(dibenzylideneacetone)palladium (0) (0.36 g, 0.62 mmol), 2,4,6-triisopropyl-2'-(dicyclohexylphosphino)biphenyl (0.59 g, 1.24 mmol) and potassium acetate (3.66 g, 37.3 mmol) were added uder nitrogen atmosphere, and stirred at 65°C for 16 h. The reaction mixture was diluted with ethyl acetate (200 mL), filtered through Celite pad to remove insoluble material. Water (200 mL) was added to the filtrate, and extracted with ethyl acetate (2x200 mL). The combined organic layer was washed with brine, dried over sodium sulfate, filtered and concentrated. The crude material was purified by chromatography on silica gel, eluted with hexane/ethyl acetate. To the oily material, hexane

was added, and then the precipitate was collected by filtration to afford 2-(6-cyclopropyl-8-fluoro-1-oxoisoquinolin-2(1*H*)-yl)-6-(4,4,5, 5-tetramethyl-1,3,2-dioxaborolan-2-yl)benzyl acetate (3.05 g).

$^1$H NMR (400 MHz, $CDCl_3$) δ7.93 (dd, J = 7.4, 1.4 Hz, 1H), 7.47 (t, J = 7.6 Hz, 1H), 7.35 (dd, J = 7.8, 1.5 Hz, 1H), 7.02 (d, J = 7.4 Hz, 1H), 7.00 (d, J = 1.6 Hz, 1H), 6.78 (dd, J = 12.7, 1.7 Hz, 1H), 6.40 (dd, J = 7.4, 2.1 Hz, 1H), 5.45 (d, J = 11.8 Hz, 1H), 5.03 (d, J = 11.9 Hz, 1H), 2.03 - 1.93 (m, 1H), 1.92 (s, 3H), 1.34 (s, 12H), 1.15 - 1.08 (m, 2H), 0.87 - 0.80 (m, 2H); LCMS (m/z): 478.2 $[M+H]^+$.

(Eighth Step)

A solution of 4-amino-2,6-dichloropyrimidine (736 mg, 4.49 mmol) and 4-morpholinoaniline (400 mg, 2.24 mmol) in dimethoxyethane (18 mL) was heated with the microwave synthesizer at 1204°C for 12 h. The reaction mixture was cooled to ambient temperature, filtered to remove insoluble material, water was added to the filtrate and then extracted with ethyl acetate. The organic layer was washed with water and brine, dried over sodium sulfate, filtered and concentrated. The crude material was purified by chromatography on silica gel, eluted with hexane/ethyl acetate to afford 6-chloro-$N^2$-(4-morpholinophenyl)pyrimidine-2,4-diamine (218 mg).

$^1$H NMR (400 MHz, DMSO-$d_6$) δ 9.06 (s, 1H), 7.61 - 7.50 (m, 2H), 6.89 - 6.79 (m, 4H), 5.84 (s, 1H), 3.76 - 3.69 (m, 4H), 3.05 - 2.97 (m, 4H); LCMS (m/z): 306.1 $[M+H]^+$.

(Ninth Step)

To a stirred solution of 2-(6-cyclopropyl-8-fluoro-1-oxoisoquinolin-2(1*H*)-yl)-6-(4,4,5, 5-tetramethyl-1,3,2-dioxaborolan-2-yl)benzyl acetate (54 mg, 0.11 mmol) which was afforded in the Seventh Step and 6-chloro-$N^2$-(4-morpholinophenyl)pyrimidine-2,4-diamine (35 mg, 0.11 mmol) in dimethoxyethane (1.7 mL), tetrakis(triphenylphosphine)palladium (0) (13.2 mg, 0.011 mmol) and potassium carbonate (32 mg, 0.23 mmol) in water solution (0.57 mL) were added then heated with the microwave synthesizer at 110°C for 10 min. Water was added to the reaction mixture, and extracted with ethyl acetate, the organic layer was washed with water and brine, dried over sodium sulfate, filtered and concentrated. The crude material was purified by chromatography on silica gel, eluted with hexane/ethyl acetate to afford a mixture of 2-(3-{6-amino-2-[(4-morpholinophenyl)amino]pyrimidin-4-yl}-2-( hydroxymethyl)phenyl)-6-cyclopropyl-8-fluoroisoquinolin-1(2H)-one and its acetylate. The mixed material was dissolved in methanol (2 mL), potassium carbonate (100 mg, 0.724 mmol) was added and stirred at ambient temperature for 2 h. The reaction mixture was diluted with water, extracted with ethyl acetate, then the organic layer was washed with water and brine, dried over sodium sulfate, filtered and concentrated to afford the titled compound (23 mg).

$^1$H NMR (400 MHz, DMSO-$d_6$) δ8.87 (s, 1H), 7.62 - 7.48 (m, 4H), 7.43 - 7.24 (m, 3H), 6.99 (dd, J = 13.2, 1.7 Hz, 1H), 6.88 - 6.79 (m, 2H), 6.74 (s, 2H), 6.60 (dd, J = 7.5, 2.1 Hz, 1H), 6.09 (s, 1H), 5.03 - 4.95 (m, 1H), 4.33 - 4.24 (m, 1H), 4.11 - 4.00 (m, 1H), 3.76 - 3.69 (m, 4H), 3.04 - 2.97 (m, 4H), 2.13 - 2.02 (m, 1H), 1.14 - 1.03 (m, 2H), 0.91 - 0.82 (m, 2H) LCMS (m/z): 579.1 $[M+H]^+$.

Example 2

2-(3-{6-Amino-2-[(1-methyl-1H-pyrazol-4-yl)amino]pyrimidin-4-y 1}-2-(hydroxymethyl)phenyl)-6-cyclopropyl-8-fluoroisoquinolin-1(2*H*)-one

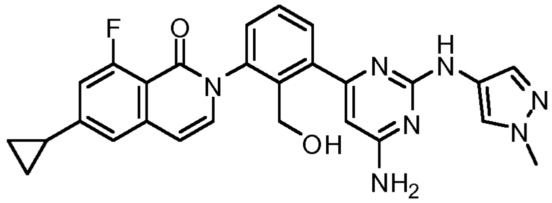

(First Step)

To a solution of 4-amino-2, 6-dichloropyrimidine (469 mg, 2.86 mmol) and 1-methyl-1*H*-pyrazol-4-amine (139 mg, 1.43 mmol) in ethanol (2.8 mL), 2 M hydrochloric acid solution (2 drops) was added and then stirred at 80°C for 3.5 h. The reaction mixture was cooled to ambient temperature, and filtered to remove insoluble material. Water was added to the filtrate, then extracted with ethyl acetate, and then the organic layer was washed with water, saturated sodium

hydrogen carbonate solution and brine, dried over sodium sulfate, filtered and concentrated. The crude material was purified by chromatography on silica gel, eluted with hexane/ethyl acetate to afford 6-chloro-*N*2-(1-methyl-1*H*-pyrazol-4-yl)pyrimidine-2,4-diamine (40 mg).

$^1$H NMR (400 MHz, DMSO-$d_6$) δ9.16 (s, 1H), 7.87 (s, 1H), 7.44 (s, 1H), 6.97 - 6.71 (m, 2H), 5.80 (s, 1H), 3.77 (s, 3H) ; LCMS (m/z): 225.1 [M+H] $^+$.

(Second Step)

2-(6-cyclopropyl-8-fluoro-1-oxoisoquinolin-2(1*H*)-yl)-6-(4,4,5, 5-tetramethyl-1,3,2-dioxaborolan-2-yl)benzyl acetate (121 mg, 0.25 mmol) which was afforded in the Example 1, Seventh Step and 6-chloro-*N*2-(1-methyl-1*H*-pyrazol-4-yl)pyrimidine-2,4-diamine (57 mg, 0.25 mmol) which was similarly prepared according to the procedure described in the First Step were dissolved in dimethoxyethane (3.8 mL), and to this solution tetrakis(triphenylphosphine)palladium (0) (29.3 mg, 0.025 mmol) and potassium carbonate (70 mg, 0.5 mmol) in aqueous solution (1.2 mL) were added and then heated with the microwave synthesizer at 110°C for 10 min. Water was added to the reaction mixture, and extracted with ethyl acetate, then the organic layer was washed with water and brine, dried over sodium sulfate, filtered and concentrated. The crude material was purified by chromatography on silica gel, eluted with hexane/ethyl acetate to afford a mixture of 2-(3-{6-amino-2-[(1-methyl-1*H*-pyrazol-4-yl)amino]pyrimidin-4-y 1}-2-(hydroxymethyl)phenyl)-6-cyclopropyl-8-fluoroisoquinolin-1(2*H*)-one and its acetylate. The mixed material was dissolved in methanol (2 mL), potassium carbonate (100 mg, 0.724 mmol) was added and stirred at ambient temperature for 2 h. The reaction mixture was diluted with water, extracted with ethyl acetate, then the organic layer was washed with water and brine, dried over sodium sulfate, filtered and concentrated to afford the titled compound (78 mg).

$^1$H NMR (400 MHz, DMSO-$d_6$) δ8.95 (s, 1H), 7.93 (s, 1H), 7.58 - 7.44 (m, 3H), 7.44 - 7.31 (m, 2H), 7.27 (d, J = 1.6 Hz, 1H), 6.99 (dd, J = 13.3, 1.6 Hz, 1H), 6.79 (s, 2H), 6.61 (dd, J = 7.4, 2.1 Hz, 1H), 6.06 (s, 1H), 5.15 (s, 1H), 4.33 - 4.24 (m, 1H), 4.12 - 3.98 (m, 1H), 3.77 (s, 3H), 2.13 - 2.02 (m, 1H), 1.15 - 1.03 (m, 2H), 0.92 - 0.82 (m, 2H); LCMS (m/z): 498.5 [M+H] $^+$.

Example 3-26

Each of the Example compounds shown in the following [Table 1-1] to [Table 1-2] were prepared according to the procedure described in the above Examples or modified procedure well known in the art of organic chemistry if needed, using appropriate starting materials (those materials are obtained from commercial sources, or are prepared by literature procedures or modifications of literature procedures known to persons skilled in the art).

The physicochemical data of each compound were shown in the following [Table 2-1] to [Table 2-2].

[Table 1-1]

| Ex. No. | Structure | Compound Name |
|---|---|---|
| 3 | 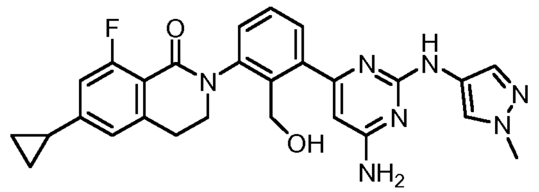 | 2-(3-{6-Amino-2-[(1-methyl -1*H*-pyrazol-4-yl)amino]pyr imidin-4-yl}-2-(hydroxymet hyl)phenyl)-6-cyclopropyl-8-fluoro-3,4-dihydroisoqui nolin-1(2*H*)-one |
| 4 | 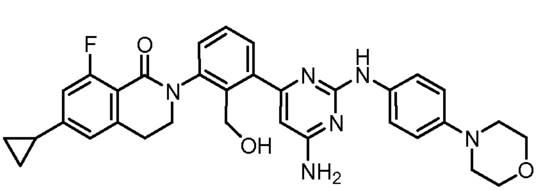 | 2-(3-{6-Amino-2-[(4-morpho linophenyl)amino]pyrimidin -4-yl}-2-(hydroxymethyl)ph enyl)-6-cyclopropyl-8-fluo ro-3,4-dihydroisoquinolin-1(2*H*)-one |

(continued)

| Ex. No. | Structure | Compound Name |
|---|---|---|
| 5 | 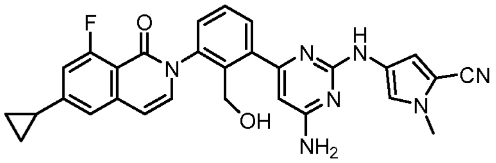 | 4-({4-Amino-6-[3-(6-cyclop ropyl-8-fluoro-1-oxoisoqui nolin-2(1*H*)-yl)-2-(hydroxy methyl)phenyl]pyrimidin-2-yl}amino)-1-methyl-1*H*-pyrr ole-2-carbonitrile |
| 6 | 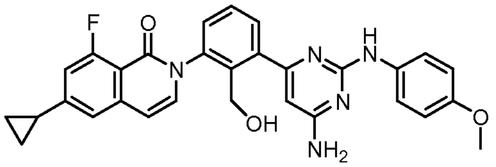 | 2-(3-{6-Amino-2-[(4-methox yphenyl)amino]pyrimidin-4-yl}-2-(hydroxymethyl)pheny l)-6-cyclopropyl-8-fluoroi soquinolin-1(2*H*)-one |
| 7 | 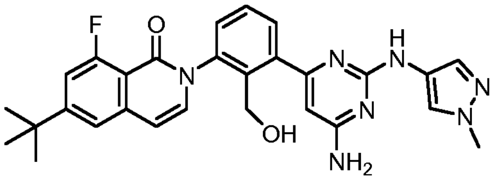 | 2-(3-{6-Amino-2-[(1-methyl -1*H*-pyrazol-4-yl)amino]pyr imidin-4-yl}-2-(hydroxymet hyl)phenyl)-6-(tert-butyl) -8-fluoroisoquinolin-1(2H) -one |
| 8 | 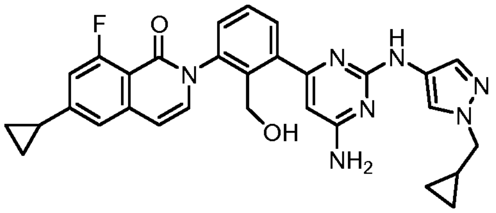 | 2-[3-(6-Amino-2-{[1-(cyclo propylmethyl)-1*H*-pyrazol-4 -yl]amino}pyrimidin-4-yl)-2-(hydroxymethyl)phenyl]-6 -cyclopropyl-8-fluoroisoqu inolin-1(2*H*)-one |
| 9 | 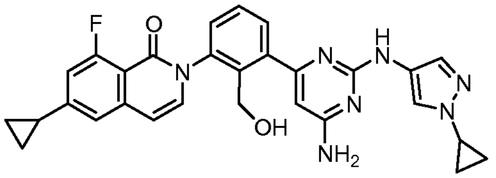 | 2-(3-{6-Amino-2-[(1-cyclop ropyl-1*H*-pyrazol-4-yl)amin o]pyrimidin-4-yl}-2-(hydro xymethyl)phenyl)-6-cyclopr opyl-8-fluoroisoquinolin-1 (2*H*)-one |
| 10 | 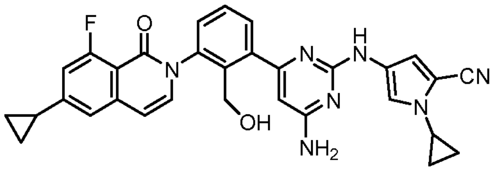 | 4-({4-Amino-6-[3-(6-cyclop ropyl-8-fluoro-1-oxoisoqui nolin-2(1*H*)-yl)-2-(hydroxy methyl)phenyl]pyrimidin-2-yl}amino)-1-cyclopropyl-1*H* -pyrrole-2-carbonitrile |
| 11 | 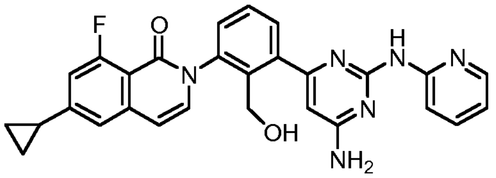 | 2-{3-[6-Amino-2-(pyridin-2 -ylamino)pyrimidin-4-yl]-2 -(hydroxymethyl)phenyl}-6-cyclopropyl-8-fluoroisoqui nolin-1(2*H*)-one |
| 12 | 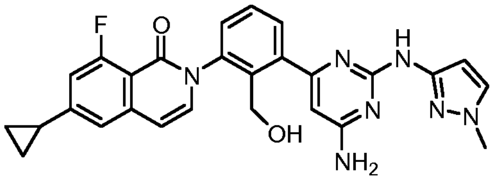 | 2-(3-{6-Amino-2-[(1-methyl -1*H*-pyrazol-3-yl)amino]pyr imidin-4-yl}-2-(hydroxymet hyl)phenyl)-6-cyclopropyl-8-fluoroisoquinolin-1(2H)-one |

(continued)

| Ex. No. | Structure | Compound Name |
|---|---|---|
| 13 | 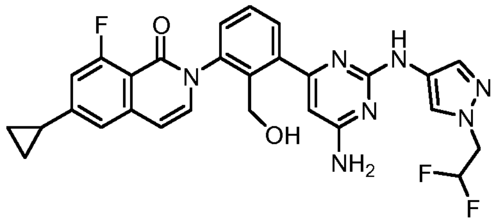 | 2-[3-(6-Amino-2-{[1-(2,2-d ifluoroethyl)-1*H*-pyrazol-4 -yl]amino}pyrimidin-4-yl)-2-(hydroxymethyl)phenyl]-6 -cyclopropyl-8-fluoroisoqu inolin-1(2*H*)-one |
| 14 | 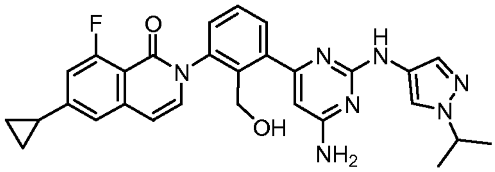 | 2-(3-{6-Amino-2-[(1-isopro pyl-1*H*-pyrazol-4-yl)amino] pyrimidin-4-yl}-2-(hydroxy methyl)phenyl)-6-cycloprop yl-8-fluoroisoquinolin-1(2 *H*)-one |

[Table 1-2]

| | | |
|---|---|---|
| 15 | 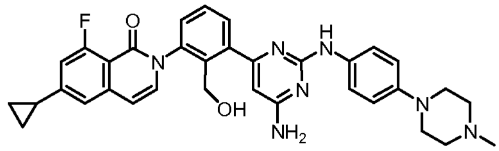 | 2-[3-(6-Amino-2-{[4-(4-meth ylpiperazin-1-yl)phenyl]ami no}pyrimidin-4-yl)-2-(hydro xymethyl)phenyl]-6-cyclopro pyl-8-fluoroisoquinolin-1(2 *H*)-one |
| 16 | 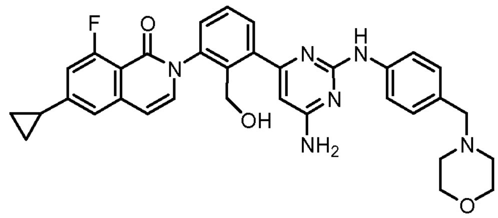 | 2-[3-(6-Amino-2-{[4-(morpho linomethyl)phenyl]amino}pyr imidin-4-yl)-2-(hydroxymeth yl)phenyl]-6-cyclopropyl-8-fluoroisoquinolin-1(2H)-one |
| 17 | 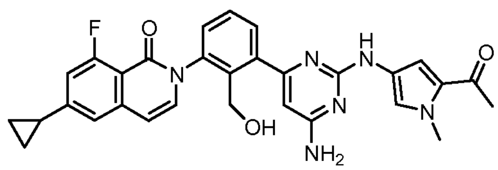 | 2-(3-{2-[(5-Acetyl-1-methyl -1*H*-pyrrol-3-yl)amino]-6-am inopyrimidin-4-yl}-2-(hydro xymethyl)phenyl)-6-cyclopro pyl-8-fluoroisoquinolin-1(2 *H*)-one |
| 18 | 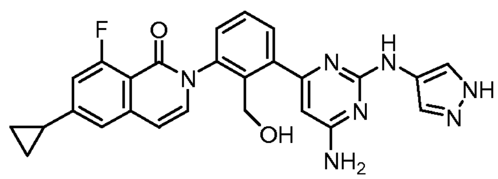 | 2-(3-{2-[(1*H*-Pyrazol-4-yl)a mino]-6-aminopyrimidin-4-yl }-2-(hydroxymethyl)phenyl)-6-cyclopropyl-8-fluoroisoqu inolin-1(2*H*)-one |
| 19 | 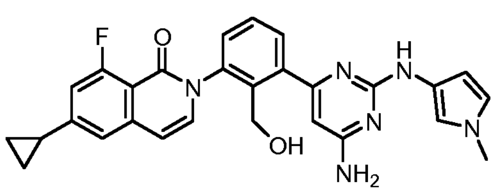 | 2-(3-{6-Amino-2-[(1-methyl-1*H*-pyrrol-3-yl)amino}pyrimi din-4-yl}-2-(hydroxymethyl) phenyl)-6-cyclopropyl-8-flu oroisoquinolin-1(2*H*)-one |

(continued)

| | | |
|---|---|---|
| 20 | 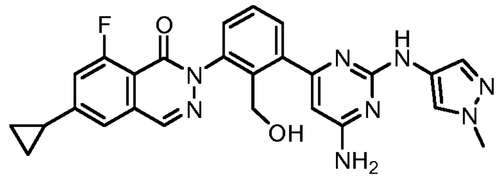 | 2-(3-{6-Amino-2-[(1-methyl-1*H*-pyrazol-4-yl)amino]pyrim idin-4-yl}-2-(hydroxymethyl )phenyl)-6-cyclopropyl-8-fl uorophthalazin-1(2*H*)-one |
| 21 | 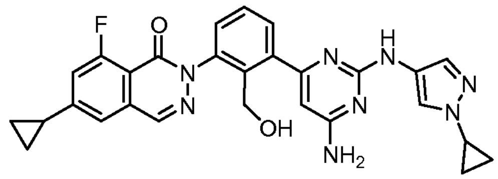 | 2-(3-{6-Amino-2-[(1-cyclopr opyl-1*H*-pyrazol-4-yl)amino] pyrimidin-4-yl}-2-(hydroxym ethyl)phenyl)-6-cyclopropyl -8-fluorophthalazin-1(2H)-o ne |
| 22 | 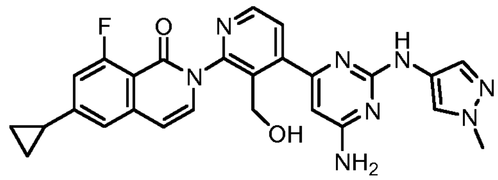 | 2-(4-{6-Amino-2-[(1-methyl-1*H*-pyrazol-4-yl)amino]pyrim idin-4-yl}-3-(hydroxymethyl )pyridin-2-yl)-6-cyclopropy 1-8-fluoroisoquinolin-1(2*H*) -one |
| 23 | 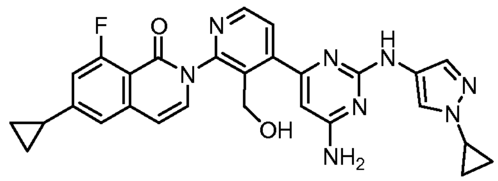 | 2-(4-{6-Amino-2-[(1-cyclopr opyl-1*H*-pyrazol-4-yl)amino] pyrimidin-4-yl}-3-(hydroxym ethyl)pyridin-2-yl)-6-cyclo propyl-8-fluoroisoquinolin-1(2*H*)-one |
| 24 | 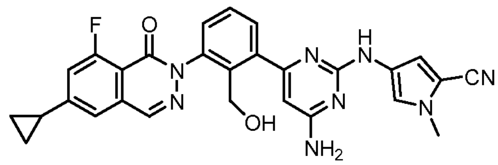 | 4-({4-Amino-6-[3-(6-cyclopr opyl-8-fluoro-1-oxophthalaz in-2(1*H*)-yl)-2-(hydroxymeth yl)phenyl]pyrimidin-2-yl}am ino)-1-methyl-1*H*-pyrrole-2-carbonitrile |
| 25 | 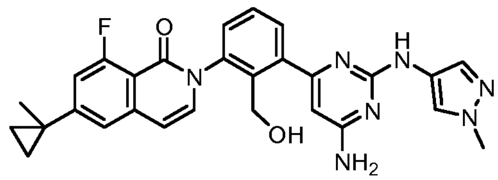 | 2-(3-{6-Amino-2-[(1-methyl-1*H*-pyrazol-4-yl)amino]pyrim idin-4-yl}-2-(hydroxymethyl )phenyl)-8-fluoro-6-(1-meth ylcyclopropyl)isoquinolin-1 (2*H*)-one |
| 26 | 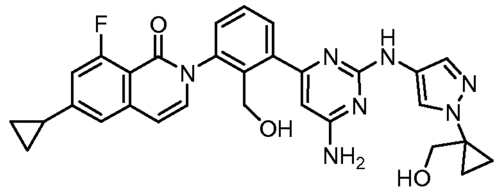 | 2-{3-[6-Amino-2-({1-[1-(hyd roxymethyl)cyclopropyl]-1*H*-pyrazol-4-yl}amino)pyrimidi n-4-yl]-2-(hydroxymethyl)ph enyl}-6-cyclopropyl-8-fluor oisoquinolin-1(2*H*)-one |

[Table 2-1]

| Ex. No. | $^1$H-NMR δ (ppm) | LCMS m/z $[M+H]^+$ |
|---|---|---|
| 3 | (DMSO-d6) δ8.95 (s, 1H), 7.93 (s, 1H), 7.52 - 7.34 (m, 4H), 6. 95 (d, J = 1.6 Hz, 1H), 6.88 (dd, J = 12. 8, 1 .7 Hz, 1H), 6.77 (s, 2H), 6.05 (s, 1H), 5.14 (s, 1H), 4.40 - 4.22 (m, 2H), 3. 93 - 3.84 (m, 1H), 3.83 - 3.75 (m, 4H), 3.26 - 3.15 (m, 1H), 3.12 - 3.00 (m, 1H), 2.05 - 1.93 (m, 1H), 1.10 - 0.99 (m, 2H), 0.85 - 0.75 (m, 2H). | 500.3 |

(continued)

| Ex. No. | $^1$H-NMR δ (ppm) | LCMS m/z $[M+H]^+$ |
|---|---|---|
| 4 | (DMSO-d6) δ8.86 (s, 1H), 7.60 - 7.54 (m, 2H), 7.49 -7.36 (m, 3H), 6.94 (d, J = 1.6 Hz, 1H), 6.88 (dd, J = 12.7, 1.7 Hz, 1H), 6.86 - 6.81 (m, 2H), 6.72 (s, 2H), 6.08 (s, 1H), 5.05 - 4.94 (m, 1H), 4.37 - 4.24 (m, 2H), 3.93 - 3.83 (m, 1H), 3.82 - 3.68 (m, 5H), 3.25 - 3.15 (m, 1H), 3.11 - 2.96 (m, 5H), 2.05 - 1.93 (m, 1H), 1.11 - 1.00 (m, 2H), 0.85 - 0.75 (m, 2H). | 581.4 |
| 5 | (DMSO-d6) δ9.08 (s, 1H), 7.67 - 7.48 (m, 3H), 7.45 - 7.31 (m, 2H), 7.30 - 7.24 (m, 1H), 6.99 (dd, J = 13.2, 1.7 Hz, 1H), 6.90 (s, 1H), 6.86 - 6.81 (m, 2H), 6.61 (dd, J = 7.4, 2.1 Hz, 1H), 6.08 (s, 1H), 5.11 (s, 1H), 4.33 - 4.25 (m, 1H), 4.13 - 4.01 (m, 1H), 3.70 (s, 3H), 2.13 - 2.02 (m, 1H), 1.15 - 1.01 (m, 2H), 0.92 - 0.83 (m, 2H). | 522.6 |
| 6 | (DMSO-d6) δ8.91 (s, 1H), 7.64 - 7.57 (m, 2H), 7.56 - 7.51 (m, 2H), 7.41 - 7.36 (m, 1H), 7.32 (dd, J = 7.4, 0.9 Hz, 1H), 7.27 (d, J = 1.7 Hz, 1H), 6.99 (dd, J = 13.2, 1.7 Hz, 1H), 6.84 - 6.78 (m, 2H), 6.78 - 6.69 (m, 2H), 6.60 (dd, J = 7.5, 2.2 Hz, 1H), 6.10 (s, 1H), 5.06 - 4.90 (m, 1H), 4.32 - 4.25 (m, 1H), 4.11 - 4.00 (m, 1H), 3.70 (s, 3H), 2.13 - 2.02 (m, 1H), 1 . 14 - 1.06 (m, 2H), 0.92 - 0.82 (m, 2H). | 524.2 |
| 7 | (DMSO-d6) δ8.96 (s, 1H), 7.93 (s, 1H), 7.58- 7.51 (m, 3H), 7.46 (s, 1H), 7.42 - 7.30 (m, 3H), 6.87 - 6.74 (m, 2H), 6.73 - 6.64 (m, 1H), 6.06 (s, 1H), 5.15 (s, 1H), 4.37 - 4.22 (m, 1H), 4.18 - 4.00 (m, 1H), 3.77 (s, 3H), 1.35 (s, 9H). | 514.2 |
| 8 | (DMSO-d6) δ9.08 - 8.70 (m, 1H), 8.07 - 7.94 (m, 1H), 7.58 - 7.50 (m, 2H), 7.50 - 7.46 (m, 1H), 7.39 (dd, J = 7.1, 2.1 Hz, 1H), 7.35 - 7.32 (m, 1H), 7.29 - 7.26 (m, 1H), 7.04 - 6.95 (m, 1H), 6.90 - 6.67 (m, 2H), 6.66 - 6.56 (m, 1H), 6.07 (s, 1H), 5.34 - 4.95 (m, 1H), 4.40 - 4.23 (m, 1H), 4.15 - 4.00 (m, 1H), 3.97 - 3.83 (m, 2H), 2.13 - 2.02 (m, 1H), 1.27 - 1.15 (m, 1H), 1.14 - 1.05 (m, 2H), 0.92 - 0.83 (m, 2H), 0.57 - 0.47 (m, 2H), 0.40 - 0.29 (m, 2H). | 538.3 |
| 9 | (DMSO-d6) δ8.96 (s, 1H), 7.99 (s, 1H), 7.56 - 7.52 (m, 2H), 7.42 (s, 1H), 7.39 (dd, J = 6.3, 2.9 Hz, 1H), 7.33 (d, J = 7.4 Hz, 1H), 7.27 (d, J = 1.6 Hz, 1H), 6.99 (dd, J = 13.2, 1.6 Hz, 1H), 6.93 - 6.69 (m, 2H), 6.61 (dd, J = 7.5, 2.1 Hz, 1H), 6.06 (s, 1H), 5.15 (s, 1H), 4.34 - 4.22 (m, 1H), 4.14 - 4.01 (m, 1H), 3.66 - 3.57 (m, 1H), 2.12 - 2.02 (m, 1H), 1.14 - 1.06 (m, 2H), 1.04 - 0.96 (m, 2H), 0.95 - 0.81 (m, 4H). | 524.2 |
| 10 | (DMSO-d6) δ9.04 (s, 1H), 7.57 - 7.52 (m, 2H), 7.46 (s, 1H), 7.39 (dd, J = 5.9, 3.3 Hz, 1H), 7.33 (d, J = 7.4 Hz, 1H), 7.27 (d, J = 1.6 Hz, 1H), 6.99 (dd, J = 13.2, 1.7 Hz, 1H), 6.96 - 6.77 (m, 3H), 6.61 (dd, J = 7.5, 2.1 Hz, 1H), 6.08 (s, 1H), 5.09 (s, 1H), 4.37 - 4.22 (m, 1H), 4.14 - 4.02 (m, 1H), 3.54 - 3.45 (m, 1H), 2.13 - 2.02 (m, 1H), 1.14 - 1.06 (m, 2H), 1.06 - 0.96 (m, 4H), 0.90 - 0.84 (m, 2H). | 548.2 |
| 11 | (DMSO-d6) δ9.43 (s, 1H), 8.30 - 8.16 (m, 2H), 7.72 - 7.50 (m, 3H), 7.50 - 7.32 (m, 2H), 7.30 - 7.25 (m, 1H), 7.03 - 6.87 (m, 4H), 6.61 (dd, J = 7.5, 2.1Hz, 1H), 6.25 (s, 1H), 5.19-5.06 (m, 1H), 4.35 - 4.26 (m, 1H), 4.13 - 4.00 (m, 1H), 2.14 - 2.02 (m, 1H), 1.22 - 1.01 (m, 2H), 0.96 - 0.79 (m, 2H). | 495.0 |
| 12 | (DMSO-d6) δ9.25 (s, 1H), 7.55 - 7.51 (m, 2H), 7.48 (d, J = 2.2 Hz, 1H), 7.41 - 7.37 (m, 1H), 7.34 (d, J = 7.4 Hz, 1H), 7.27 (d, J = 1 . 7 Hz, 1H), 6.99 (dd, J = 13.2, 1.7 Hz, 1H), 6.84 - 6.68 (m, 2H), 6.61 (dd, J = 7.5, 2.1 Hz, 1H), 6.51 (s, 1H), 6.10 (s, 1H), 5.27 - 5.14 (m, 1H), 4.24 (dd, J = 11.9, 4.5 Hz, 1H), 4.03 - 3.95 (m, 1H), 3.69 (s, 3H), 2.13 - 2.03 (m, 1H), 1.13 - 1.07 (m, 2H), 0.92 - 0.82 (m, 2H). | 498.3 |
| 13 | (DMSO-d6) δ 9.04 (s, 1H), 8.01 (s, 1H), 7.63 (s, 1H), 7.58 - 7.50 (m, 2H), 7.39 (dd, J = 6.9, 2.3 Hz, 1H), 7.34 (d, J = 7.3 Hz, 1H), 7.27 (d, J = 1.6 Hz, 1H), 6.99 (dd, J = 13.2, 1.7 Hz, 1H), 6.92 - 6.68 (m, 2H), 6.60 (dd, J = 7.4, 2.1 Hz, 1H), 6.32 (tt, J = 55.2, 4.0 Hz, 1H), 6.09 (s, 1H), 5.12 (s, 1H), 4.51 (td, J = 15.0, 3.9 Hz, 2H), 4.36 - 4.24 (m, 1H), 4.15 - 4.03 (m, 1H), 2.13 - 2.03 (m, 1H), 1.14 - 1.06 (m, 2H), 0.91 - 0.83 (m, 2H). | 548.3 |

(continued)

| Ex. No. | $^1$H-NMR δ (ppm) | LCMS m/z [M+H]$^+$ |
|---|---|---|
| 14 | (DMSO-d6) δ8.94 (s, 1H), 7.98 (s, 1H), 7.57- 7.50 (m, 2H), 7.45 (s, 1H), 7.39 (dd, J = 6.8, 2.4 Hz, 1H), 7.33 (d, J = 7.4 Hz, 1H), 7.27 (d, J = 1.6 Hz, 1H), 6.99 (dd, J = 13.2, 1.7 Hz, 1H), 6.92 - 6.68 (m, 2H), 6.60 (dd, J = 7.5, 2.1 Hz, 1H), 6.06 (s, 1H), 5.16 (s, 1H), 4.44 - 4.34 (m, 1H), 4.33 - 4.23 (m, 1H), 4.13 - 4.01 (m, 1H), 2.13 - 2.03 (m, 1H), 1.39 (d, J = 6.6 Hz, 6H), 1.13 - 1.06 (m, 2H), 0.90 - 0.84 (m, 2H). | 526.3 |

[Table 2-2]

| | | |
|---|---|---|
| 15 | (DMSO-d6) δ8.85 (s, 1H), 7.56 - 7.51 (m, 4H), 7.38 (dd, J = 5.4, 3.8 Hz, 1H), 7.32 (d, J = 7.4 Hz, 1H), 7.27 (d, J = 1.7 Hz, 1H), 6. 99 (dd, J = 13.2, 1.7 Hz, 1H), 6.85 - 6.79 (m, 2H), 6.78 - 6.67 (m, 2H), 6.60 (dd, J = 7.5, 2.1 Hz, 1H), 6.08 (s, 1H), 5. 00 (dd, J = 8.1, 4.3 Hz, 1H), 4.28 (dd, J = 11.8, 4.2 Hz, 1H), 4.06 (dd, J = 11.7, 8.2 Hz, 1H), 3.06 - 3.00 (m, 4H), 2.47 - 2.41 (m, 4H), 2.21 (s, 3H), 2.13 - 2.03 (m, 1H), 1.14 - 1.06 (m, 2H), 0.91 - 0.83 (m, 2H). | 592.2 |
| 16 | (DMSO-d6) δ8.29 (s, 1H), 7.44 - 7.14 (m, 6H), 7.02 - 6.93 (m, 1H), 6.71 - 6.51 (m, 3H), 6.41 (dd, J = 8.2, 3.1 Hz, 2H), 6.36 - 6.14 (m, 2H), 5.05 - 4.53 (m, 2H), 4.53 - 4.34 (m, 1H), 4.12 - 3.94 (m, 2H), 3.64 - 3.53 (m, 8H), 2.12 - 2.01 (m, 1H), 1.13 - 1.05 (m, 2H), 0.91 - 0.82 (m, 2H). | 593.2 |
| 17 | (DMSO-d6) δ9.01 (s, 1H), 7.58 - 7.50 (m, 3H), 7.39 (dd, J = 6.6, 2.6 Hz, 1H), 7.34 (d, J = 7.4 Hz, 1H), 7.27 (d, J = 1.7 Hz, 1H), 7.02 - 6.94 (m, 2H), 6.92 - 6.71 (m, 2H), 6.61 (dd, J = 7.5, 2.1 Hz, 1H), 6.07 (s, 1H), 5.13 (s, 1H), 4.39 - 4.23 (m, 1H), 4.16 - 3.99 (m, 1H), 3.81 (s, 3H), 2.32 (s, 3H), 2.13 - 2.02 (m, 1H), 1 . 14 - 1.05 (m, 2H), 0.91 - 0.83 (m, 2H). | 539.2 |
| 18 | (DMSO-d6) δ 12.38 (s, 1H), 8.96 (s, 1H), 7.96 (s, 1H), 7.59 - 7.48 (m, 3H), 7.41 - 7.37 (m, 1H), 7.36 - 7.31 (m, 1H), 7.30 - 7.25 (m, 1H), 7.03 - 6.94 (m, 1H), 6.92 - 6.70 (m, 2H), 6.61 (dd, J = 7.5, 2.1 Hz, 1H), 6.05 (s, 1H), 5.19 (s, 1H), 4.31 - 4.23 (m, 1H), 4.11 - 4.00 (m, 1H), 2.13 - 2.02 (m, 1H), 1.15 - 1.03 (m, 2H), 0.92 - 0.78 (m, 2H). | 484.6 |
| 19 | (DMSO-d6) δ 8.95 - 8.51 (m, 1H), 7.60 - 7.49 (m, 2H), 7.43 - 7.36 (m, 1H), 7.33 (d, J = 7.4Hz, 1H), 7.27 (d, J = 1.7 Hz, 1H), 7.21-7.04 (m, 1H), 6.99 (dd, J = 13.2, 1.7 Hz, 1H), 6.84 - 6.64 (m, 2H), 6. 61 (dd, J = 7.5, 2. 1 Hz, 1H), 6.48 (s, 1H), 6.10 - 5.90 (m, 2H), 5.38 - 4.99 (m, 1H), 4.30 - 4.21 (m, 1H), 4.11 - 3.99 (m, 1H), 3.55 (s, 3H), 2.12 - 2.03 (m, 1H), 1.14 - 1.06 (m, 2H), 0.91 - 0.83 (m, 2H). | 497.3 |
| 20 | (DMSO-d6) δ8.94 (s, 1H), 8.60 - 8.23 (m, 1H), 8.13 - 7.74 (m, 1H), 7.78 - 7.19 (m, 6H), 7.11 - 6.46 (m, 2H), 6.02 (s, 1H), 5.08 (s, 1H), 4.53 - 4.04 (m, 2H), 3.77 (s, 3H), 2.26 - 2.09 (m, 1H), 1.29 - 1.06 (m, 2H), 1.02 - 0.83 (m, 2H). | 499.2 |
| 21 | (DMSO-d6) δ8.94 (s, 1H), 8.54 - 8.29 (m, 1H), 7.99 (s, 1H), 7.70 - 7.28 (m, 6H), 7.04 - 6.56 (m, 2H), 6.03 (s, 1H), 4.46 - 4.10 (m, 2H), 3.72 - 3.48 (m, 1H), 2.25 - 2.08 (m, 1H), 1.30 - 1.09 (m, 4H), 1.08 - 0.75 (m, 4H). | 525.2 |
| 22 | (DMSO-d6) δ8.77 - 8.42 (m, 1H), 8.17 - 7.70 (m, 1H), 7.73 - 7.17 (m, 4H), 7.14 - 6.75 (m, 3H), 6.75 - 6.50 (m, 1H), 6.10 (s, 1H), 4.62 - 4.39 (m, 1H), 4.36 - 4.14 (m, 1H), 3.77 (s, 3H), 2.16 - 2.02 (m, 1H), 1.16 - 1.04 (m, 2H), 0.94 - 0.80 (m, 2H). | 498.9 |
| 23 | (DMSO-d6) δ8.79 - 8.48 (m, 1H), 8.20 - 7.90 (m, 1H), 7.92 - 7.71 (m, 1H), 7.72 - 7.37 (m, 4H), 7.37 - 7.21 (m, 1H), 7.17 - 6.88 (m, 1H), 6.64 (dd, J = 7.4, 2.1 Hz, 1H), 6.12 (s, 1H), 4.65 - 4.40 (m, 1H), 4.37 - 4.16 (m, 1H), 3.72 - 3.56 (m, 1H), 2.17 - 1.98 (m, 1H), 1.43 - 0.65 (m, 8H). | 525.3 |
| 24 | (DMSO-d6) δ9.04 (s, 1H), 8.41 (d, J = 2.5 Hz, 1H), 7.56 (d, J = 1.6 Hz, 1H), 7.54 - 7.46 (m, 3H), 7.46 - 7.39 (m, 2H), 6. 95 - 6.67 (m, 3H), 6.04 (s, 1H), 5. 17 - 4.88 (m, 1H), 4.39 - 4.15 (m, 2H), 3.70 (s, 3H), 2.23 - 2.13 (m, 1H), 1.21 - 1.12 (m, 2H), 0.97 - 0.89 (m, 2H). | 523.2 |
| 25 | (DMSO-d6) δ7.94 (s, 1H), 7.65 - 7.28 (m, 6H), 7.12 - 6.98 (m, 1H), 6.90 - 6.72 (m, 2H), 6.74 - 6.59 (m, 1H), 6.06 (s, 1H), 4.39 - 4.19 (m, 1H), 4.18 - 3.94 (m, 1H), 3.77 (s, 3H), 1.47 (s, 3H), 1.10 - 0.97 (m, 2H), 0.98 - 0.85 (m, 2H). | 512.1 |

(continued)

| | | |
|---|---|---|
| 26 | (DMSO-d6) δ8.93 (br, 1H), 7.94 (s, 1H), 7.61 - 7.45 (m, 3H), 7.39 (dd, J= 7.1, 2.1 Hz, 1H), 7.33 (d, J = 7.4 Hz, 1H), 7.27 (s, 1H), 6.99 (dd, J = 13.2, 1.7 Hz, 1H), 6.82 (br, 2H), 6.61 (dd, J = 7.5, 2.1 Hz, 1H), 6.08 (s, 1H), 5.18 (br, 1H), 4.89 (br, 1H), 4.38 - 4.19 (m, 1H), 4.18 - 3.98 (m, 1H), 3.62 (s, 2H), 2.16 - 1.99 (m, 1H), 1.16 - 1.04 (m, 4H), 1.02 - 0.94 (m, 2H), 0.92 - 0.81 (m, 2H). | 554.2 |

Example 27

2-(3-{6-Amino-2-[(1-methyl-1*H*-pyrazol-4-yl)amino]pyrimidin-4-y 1}-2-(hydroxymethyl)phenyl)-6-cyclopropyl-8-fluoroisoquinolin-1(2*H*)-one hydrochloride

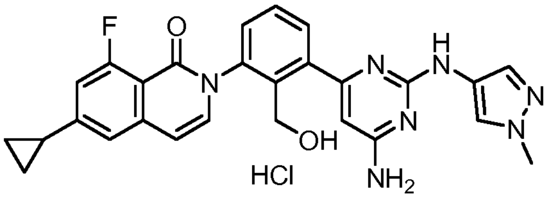

To a stirred suspension of 2-(3-{6-amino-2-[(1-methyl-1*H*-pyrazol-4-yl)amino]pyrimidin-4-y 1}-2-(hydroxymethyl)phenyl)-6-cyclopropyl-8-fluoroisoquinolin-1(2*H*)-one (100 mg, 0.20 mmol) which afforded in the Example 2 in ethanol (2 mL), 2 M solution of hydrogen chloride in ethanol (0.1 mL, 0.20 mmol) was added at ambient temperature and stirred for 1 h. Ethyl acetate was added to the reaction mixture, and then the precipitate was collected by filtration to afford the titled compound (73 mg).

$^1$H NMR (400 MHz, DMSO-$d_6$) δ 13.00 - 11.91 (m, 1H), 9.80 - 9.44 (m, 1H), 8.75 - 8.15 (m, 2H), 8.12 - 7.97 (m, 1H), 7.72 - 7.51 (m, 4H), 7.37 - 7.24 (m, 2H), 7.01 (dd, J = 13.2, 1.7 Hz, 1H), 6.64 (dd, J = 7.5, 2.1 Hz, 1H), 6.25 (s, 1H), 5.74 - 4.72 (m, 1H), 4.37 (d, J = 12.4 Hz, 1H), 4.26 (d, J = 12.5 Hz, 1H), 3.83 (s, 3H), 2.14 - 2. 03 (m, 1H), 1.16-1.05 (m, 2H), 0.92 - 0.82 (m, 2H) ; LCMS (m/z) : 498.3 $[M+H]^+$.

Test Example 1

BTK activity inhibition test

(Preparation of dephosphorylated BTK)

Dephosphorylated BTK was obtained by adding λ protein phosphatase (manufactured by New England BioLabs Inc., Code No.P0753S) and $MnCl_2$ at 10 U/μg and 2 mM, respectively to biotinylated BTK protein BTN-BTK (manufactured by Carna Biosciences, Inc.) enzyme solution, reacting the mixture at 4°C overnight, and removing of λ protein phosphatase by anti DYKDDDDK-tag antibody agarose gel chromatography, followed by buffer exchange using a 10DG Desalting Column.

(Kinase activity measuring method)

The kinase activity was measured using QuickScout Screening Assist (trade mark) MSA (commercially available kit manufactured by Carna Biosciences, Inc.) by mobility shift assay (MSA) method. The substrate of the kinase reaction was an FITC-labeled SRCtide peptide included in the kit. An assay buffer [20 mM HEPES, 0.01% Triton X-100 (Trade mark), 2 mM dithiothreitol, pH7.5] was used and adjusted at 4 μM substrate, 20 mM $MgCl_2$ and 200 μM ATP to obtain a substrate mixture solution. The enzyme solution was also prepared by diluting the dephosphorylated BTK to 0.6 nM using the assay buffer. The 10 mM solution of the test compound in DMSO was further diluted with DMSO to 10 levels of the concentration (0. 00003 mM, 0.0001 mM, 0.0003 mM, 0.001 mM, 0.003 mM, 0.01 mM, 0.03 mM, 0.1 mM, 0.3 mM, 1 mM), each of which was subjected to a 25-fold dilution with the assay buffer to obtain the drug solutions (4% DMSO solutions), 5 μL of the drug solution or a control solution (4% DMSO-assay buffer), 5 μL of the substrate mixture solution, and 10 μL of the enzyme solution were mixed in the wells of a polypropylene 384-well plate and allowed to react at room temperature for 1 hour, and then quenched by adding 60 μL of the termination buffer included in the kit.

Subsequently, the quantities of the substrates (S) and the phosphorylated substrate (P) in the reaction solution were measured using LabChip EZ Reader II system (manufactured by Caliper Life Sciences) according to the protocol of the assay kit.

(BTK Inhibiting activity evaluation method)

The heights of the peaks of the isolated substrate and the phosphorylated substrate were represented as S and P, respectively, and a blank which contained the assay buffer instead of the enzyme solution was also measured.
The inhibition rate (%) of the test compound was calculated according to the following equation.

$$\text{Inhibition rate (\%)} = (1-(C-A)/(B-A)) \times 100$$

wherein, A, B and C represent P/(P+S) of the blank well, P/(P+S) of the control well and P/(P+S) of the compound-containing well, respectively.
The $IC_{50}$ value was calculated via a regression analysis of the inhibition rate (%) and the test compound concentration (logarithmic value).

(Evaluation results)

The $IC_{50}$ values of the compounds of the present invention against dephosphorylated BTK were 1 μM or less, therefore the compounds of the present invention were revealed to have potent inhibiting activity. The inhibiting activities against dephosphorylated BTK of the typical compounds were shown in the following Table 3.

[Table 3]

| Test compound (Example No.) | Dephosphorylated BTK $IC_{50}$ (nM) |
|---|---|
| 1 | 2.1 |
| 2 | 6.4 |
| 5 | 1.7 |
| 7 | 0.3 |
| 8 | 0.3 |
| 9 | 0.3 |
| 10 | 2.1 |
| 15 | 0.4 |
| 17 | 0.8 |
| 18 | 0.6 |
| 20 | 0.6 |
| 21 | 0.4 |
| 22 | 0.8 |
| 24 | 0.4 |
| 25 | 0.2 |

Test Example 2

Intracellular BTK auto-phosphorylation activity inhibition test

(Culture of cells to be used)

Ramos cells (2G6.4C10, ATCC No.CRL-1923) were cultured in a T75 flask containing RPMI-1640 medium (GIBCO, #A10491-01) supplemented with 10% FBS (AusGene) and 5% penicillin-streptomycin (Nacalai Tesque, Inc.)

(hereinafter referred to as growth medium) in a 5% $CO_2$ incubator.

(Addition of the compound to be tested)

The cultured Ramos cells were diluted to a cell density of $7.5\times10^6$ cells/mL with a serum-free RPMI-1640 (hereinafter referred to as medium) and kept at 37°C for 45 minutes. The cell suspension was dispensed in 1 mL aliquots into 2.0-mL tubes. The 0.3 mM solution of the test substance in DMSO was diluted with the medium to make a 0.9 μM test compound solution, 500 μL of which was then added to the tubes and the incubation was conducted at 37°C for 1 hour in the presence of the test compound at a final concentration of 0.3 μM. Thereafter, the anti-IgM antibody (Invitrogen, H15100) which had been diluted with the medium was added at a final concentration of 10 μg/mL, and the incubation was conducted at 37°C for 10 minutes.

(Extraction of proteins)

To the pellets obtained by recovering the cells via centrifugation, 100 μL of a Lysis buffer [RIPA Buffer(×1)(Cell Signaling Technology, Inc.) supplemented with 1% Phosphatase Inhibitor Cacktail 3 (Sigma Corporation, No.P0044), 1% Phosphatase Inhibitor Cacktail (Nacalai Tesque, Inc., No.07575) and 1 mM phenylmethylsulfonyl fluoride (PMSF)] was added and stirred gently and then allowed to stand for 10 minutes. The supernatant was recovered by centrifugation (15, 000 rpm, 15 minutes) and the protein level was quantified. The portion was mixed with the SDS-sample buffer, allowed to react for 5 minutes at 95°C to denature the protein, thereby obtaining a sample solution. Each 5 μL of the sample solutions was applied to each well containing a 4 to 20% gradient acrylamide gel (COSMO BIO Co., Ltd., No.414879) and electrophoresis was conducted. Thereafter, iBlot gel transfer system (Life Technologies Corporation) was used to transfer the proteins in the gel onto a PVDF membrane.

(Detection of BTK or phosphorylated BTK)

The PVDF membrane after transfer was blocked with 2% ECL prime blocking Reagent (GE Healthcare) and thereafter the reaction was conducted overnight at 4°C using anti-BTK mouse antibody (BD transduction laboratory, No.611116) or anti-phosphorylated BTK rabbit antibody (pY223, EPITOMICS, No.2207-1) as a primary antibody. The unreacted primary antibody was washed with a TBST buffer (10 mM Tris-HCl (pH7.5), 150 mM NaCl, 0.1% Tween 20) and then the reaction was conducted for 1 hour at room temperature in a TBST buffer supplemented with 2% ECL prime blocking Reagent using HRP-labeled anti-mouse IgG goat antibody (Life Technologies Corporation, No.62-6520) or anti-rabbit IgG goat antibody (Life Technologies Corporation, No.65-6120) as a secondary antibody. After washing the unreacted secondary antibody with the TBST buffer, ECL Prime Western Blotting Detection System (GE Healthcare) was used to conduct a reaction in accordance with the attached protocol, and then the respective bands as chemiluminescences were detected with a CCD camera (ATTO, Light-Capture II). The detected bands were subjected to densitometry (ATTO CS Analyzer ver3.0) to be represented as numerical values, and the inhibition rate (%) was calculated based on the intensity of the band in each group while taking the luminescence of the phosphorylated BTK band in the group without added compound with IgM stimulation as 100% and the luminescence of the phosphorylated BTK band in the group without added compound without IgM stimulation as 0%. Each phosphorylated BTK band was corrected based on the total BTK.

The combinations of the primary antibodies and the secondary antibodies employed in this test and the dilution magnitudes thereof are shown below.

[Table 4]

| | Primary antibody (dilution magnitude) | Secondary antibody (dilution magnitude) |
|---|---|---|
| 1 | Anti-BTK mouse antibody (1/4000) | Anti-mouse IgG goat antibody (1/5000) |
| 2 | Anti-phosphorylated BTK rabbit antibody (1/500) | Anti-rabbit IgG goat antibody (1/5000) |

The results of Test Example 2 indicate that the compounds of the present invention have potent inhibitory effects also on "the intracellular BTK autophosphorylation activity".

Test Example 3

Inhibition test on the change of Ramos intracellular calcium ion

The intracellular BTK inhibition by the compounds of the present invention was verified by measuring the effects of the compounds of the present invention on "anti-IgM antibody BCR stimulation-induced intracellular calcium influx".

(Addition of cell suspension and calcium indicator)

One day before measurement, the Ramos cells were cultured as being suspended again at a cell density of $1.0 \times 10^6$ cells/mL in a fresh growth medium (growth medium as used in Test Example 2), and the cells were recovered next day by centrifugation and washed with RPMI-1640 medium supplemented with 5% penicillin-streptomycin (Nacalai Tesque, Inc.) (Medium 1). These cells were suspended again at a cell density of $2.0 \times 10^6$ cells/mL in RPMI-1640 medium supplemented with 1% Ultra Low IgG FBS (GIBCO, #16250) and 5% penicillin-streptomycin (Nacalai Tesque, Inc.) (Medium 2), and thereafter each 100 μL of the cell suspension was added to each well of a Poly Lysine-coated microplate (BD BioCoat™, #356692), centrifuged (700 rpm, 3 minutes) and then incubated for 1 hour in a 5% $CO_2$ incubator at 37°C. Each 100 μL of a calcium indicator Fluo-8NW dye-loading solution (AAT Bioquest, #36315) was added to each well, and incubation was continued further for 30 minutes in the 5% $CO_2$ incubator at 37°C.

(Addition of the compound to be tested)

A 10 mM stock solution of a test compound in DMSO was further diluted with DMSO to 0.2 mM, and a test compound-free DMSO solution was employed as a control. Then each was subjected to a 47.6-fold dilution with Medium 2 and each 10 μL was added to each well of the aforementioned plate, which was incubated at 37°C for 10 minutes (final concentrations of the test compound: 0.2 μM).

(Measurement of calcium ion concentration)

The Ramos intracellular calcium ion concentration was measured as a fluorescent intensity of the calcium indicator Fluo-8NW using a microplate reader (SynergyH1) (Ex/Em = 490/525nm). After measuring the baseline for 15 seconds, 50 μL of the anti-IgM antibody (Invitrogen, #H15100) diluted with Medium 2 to 10.4 μg/mL was added to each well described above (final concentration of 2.0 μg/mL) to effect BCR stimulation, and then the measurement was continued further for 150 seconds.

Figure 1 shows the results of the compound of Example 1 and 2. As shown in Figure 1, the compound of the present invention inhibited "the anti-IgM antibody BCR stimulation-induced intracellular calcium ion variation" in a concentration-dependent manner from a low concentration, indicating that the BCR signal was inhibited effectively.

INDUSTRIAL APPLICABILITY

The compound provided by the present invention is useful as a preventive or therapeutic pharmaceutical (pharmaceutical composition) for diseases which are known to be involved in abnormal cell response through BTK, for example, self-immune diseases, inflammatory diseases such as allergosis, bone diseases, and cancers such as lymphoma. The compound is also useful, as a BTK inhibitor, for reagents to be used in tests and researches.

## Claims

**1.** A 2,6-diaminopyrimidine derivative represented by the following formula (I):

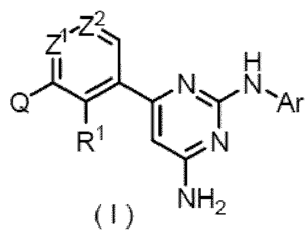

(I)

wherein

$R^1$ represents a substituted or unsubstituted lower alkyl group, or a substituted or unsubstituted alkoxy group,
Ar represents a substituted or unsubstituted aryl group, or a substituted or unsubstituted heteroaryl group,
$Z^1$ and $Z^2$ represent carbon atoms, or either 1 or 2 of the $Z^1$ and $Z^2$ represent nitrogen atoms,
Q is selected from a structure (a) or (b) described below:

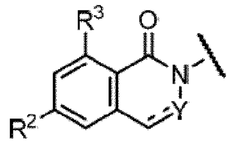
(a)

,

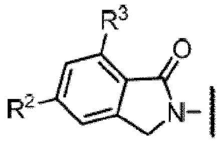
(b)

$R^2$ represents a substituted or unsubstituted lower alkyl group, or a substituted or unsubstituted cycloalkyl group,
$R^3$ represents a hydrogen atom or a halogen atom,
Y represents a nitrogen atom or a carbon atom, and

the bond drawn with a dotted line parallel to a solid line on structure (a) represents either double bond or single bond, or a pharmaceutically acceptable salt thereof.

**2.** The 2,6-diaminopyrimidine derivative according to claim 1, wherein Q is a structure (a), or a pharmaceutically acceptable salt thereof.

Fig. 1

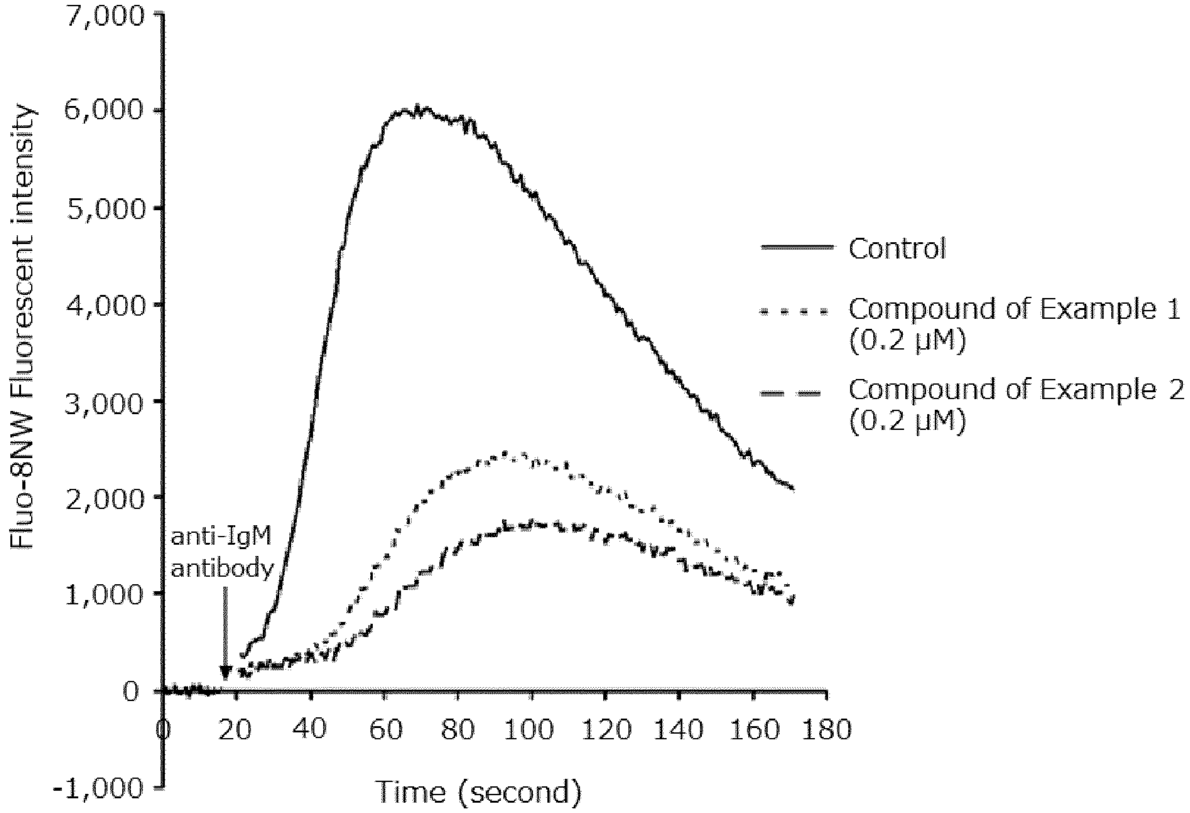
7,000
6,000
5,000
4,000
3,000
2,000
1,000
0
-1,000
Fluo-8NW Fluorescent intensity
0
20
40
60
80
100
120
140
160
180
Time (second)
anti-IgM
antibody
Control
Compound of Example 1
(0.2 μM)
Compound of Example 2
(0.2 μM)

**INTERNATIONAL SEARCH REPORT**

International application No. PCT/JP2014/072917

A. CLASSIFICATION OF SUBJECT MATTER
*C07D401/10*(2006.01)i, *C07D401/14*(2006.01)i, *A61K31/506*(2006.01)n, *A61K31/5377*(2006.01)n, *A61P19/08*(2006.01)n, *A61P29/00*(2006.01)n, *A61P35/00*(2006.01)n, *A61P37/06*(2006.01)n

According to International Patent Classification (IPC) or to both national classification and IPC

B. FIELDS SEARCHED

Minimum documentation searched (classification system followed by classification symbols)
C07D401/10, C07D401/14, A61K31/506, A61K31/5377, A61P19/08, A61P29/00, A61P35/00, A61P37/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2013/083666 A1 (F. HOFFMANN-LA ROCHE AG), 13 June 2013 (13.06.2013), claims 1 to 26; compound I-12; example 12 & US 2013/0150360 A1 | 1,2 |
| A | JP 2010-502749 A (CGI Pharmaceuticals, Inc.), 28 January 2010 (28.01.2010), claims; examples & US 2008/0125417 A1 & EP 2069314 A1 & WO 2008/033834 A1 & CL 26412007 A & AR 63946 A & CA 2661938 A & KR 10-2009-0061655 A & CN 101605766 A & IL 197231 D & ZA 200901593 A & PE 10592008 A & RU 2009113691 A & AU 2007296550 A & MX 2009002648 A & TW 200829577 A & BR PI0716888 A | 1,2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16 October, 2014 (16.10.14) | Date of mailing of the international search report<br>04 November, 2014 (04.11.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2014/072917

C (Continuation). DOCUMENTS CONSIDERED TO BE RELEVANT

| Category* | Citation of document, with indication, where appropriate, of the relevant passages | Relevant to claim No. |
|---|---|---|
| A | JP 2011-519948 A (CGI Pharmaceuticals, Inc.), 14 July 2011 (14.07.2011), claims; table 25 & US 2011/0118233 A1 & EP 2297105 A1 & WO 2009/137596 A1 & AU 2009244291 A & CA 2723237 A | 1,2 |
| A | WO 2012/135801 A1 (UNIVERSITY OF UTAH RESEARCH FOUNDATION), 04 October 2012 (04.10.2012), claims 1 to 20; table I & US 2013/0059847 A1 & EP 2694486 A1 | 1,2 |
| P,A | WO 2013/133367 A1 (Carna Biosciences, Inc.), 12 September 2013 (12.09.2013), entire text (Family: none) | 1,2 |
| P,A | WO 2013/161848 A1 (Carna Biosciences, Inc.), 31 October 2013 (31.10.2013), entire text (Family: none) | 1,2 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008033834 A
- WO 2009137596 A
- WO 2013083666 A


### Non-patent literature cited in the description


- **SATTERTHWAITE, A. B. ; WITTE, O. N.** *Immunol. Rev.,* 2000, vol. 175, 120-127
- **KUROSAKI T.** *Curr. Opin. Immunol.,* 2000, vol. 12, 276-281
- **DAVIS R. E. et al.** *Nature,* 2010, vol. 463, 88-92
- **ELLMEIER W. et al.** *FEBS J.,* 2011, vol. 278, 1990-2000
- **HALCOMB K. E.** *Mol. Immunol.,* 2008, vol. 46 (2), 233-241
- **JANSSON L. ; HOLMDAHL R.** *Clin. Exp. Immunol.,* 1993, vol. 94, 459-465
- **T. W. GREENE.** Protective Groups in Organic Synthesis. John Wiley&Sons, Inc, 1999
- **N. MIYAURA et al.** *J. Am. Chem. Soc.,* 1985, vol. 107, 972
- **N. MIYAURA ; A. SUZUKI.** *Chem. Rev.,* 1995, vol. 95, 2457